# EUROPEAN PATENT APPLICATION

(11) **EP 4 607 535 A2**
(43) Date of publication of application: **27.08.2025**
(21) Application number: 25179658.7
(22) Date of filing: 04.04.2023
(51) Int. Cl.: G16H 50/70

(54) **PLATFORMS FOR DYNAMICALLY SELECTING MESSAGES IN REAL-TIME TO USERS VIA DECISION SUPPORT TOOLS**

(30) Priority: 05.04.2022 US 202263327662 P; 06.01.2023 US 202363437456 P; 06.01.2023 US 202363437466 P
(62) Divisional of application: 23785291.8
(71) Applicant: Click Therapeutics, Inc., New York, NY 10013 (US)
(72) Inventor: MURPHY, Chris, New York, NY 10013 (US); SNIPES, Cassandra, New York, NY 10013 (US); ARORA, Manasi, New York, NY 10013 (US); KUKA, Alexander, New York, NY 10013 (US)
(74) Representative: Marks & Clerk LLP

(57) **Abstract**

Provided herein are systems and methods for dynamically providing messages based on indications of states to users at risk of migraines. A server can maintain, on a database, an identification of a set of activities for users at risk of migraines, with each activity having a set of messages to be presented in accordance with a rule. The server can receive, via a user interface, an indication identifying a state of the user associated with the migraine. The server can select, based on the state of the user identified in the response, an activity to provide to the user for the session. The server can identify, in accordance with the rule for the activity, a message from the set of messages. The server can cause the message identifying the activity to be presented via the user interface.

## Description

### CROSS REFERENCE TO RELATED APPLICATIONS

The present application claims priority to U.S. Provisional Application No. 63/327,662, filed April 5, 2022, U.S. Provisional Application No. 63/437,456, filed January 6, 2023, and U.S. Provisional Application No. 63/437,466, filed January 6, 2023, each of which are hereby incorporated by reference in their entireties.

### BACKGROUND

A migraine is a type of headache that can cause severe pain, and often includes other symptoms such as nausea, sensitivity to light and sound, and impaired judgment. Migraine attacks can last for hours to days. Certain individuals, referred to herein as "migraineurs," may experience multiple migraine headaches per month. Migraines are known to interfere with a migraineur's daily activities and responsibilities and can be ruinous to a migraineur's happiness and productivity.

Under pharmaceutical approaches, acute medications may be taken by a migraineur to lessen the severity of the pain from the migraine headache. Preventative medications also exist that help minimize the likelihood that a migraineur will experience a migraine. These medications may require prescriptions, and often require a migraineur to alone decide the correct time to take the medications, which often includes an analysis of various factors, such as whether the headache is sufficiently severe so as to justify using some of the medication and whether the migraineur might run out of the medication before the prescription can be refilled. These analyses are thus subjective to each migraineur and often performed by the migraineur while the migraineur is experiencing impaired judgement, one of the symptoms of a migraine. Moreover, many migraine medications work best when taken at the correct time. For example, certain acute medications work best when taken within the first hour or two from the onset of a migraine headache.

There may be various challenges to administering migraine medications. For example, the migraineur might make a decision about taking or not taking medications with which their prescribing physician might disagree. Yet the migraineur is often forced to make such decisions without the assistance of their prescribing physician or another healthcare provider due to, e.g., difficulties reaching them while the migraineur is experiencing a headache. For example, a migraineur likely cannot schedule an appointment and travel to the appointment while experiencing a migraine, and if the migraineur could, would likely exacerbate the migraineur's pain and other symptoms while attempting to do so. As a result, this may further lower the likelihood that migraineur would take the acute medication within two hours of the onset of the migraine headache.

Compounding these issues, obtaining rapid help such as by contacting a clinician (e.g., doctor or nurse) may be impractical especially if the clinician is unavailable at the time of the migraine attack, which can be a frustrating experience and thus increase migraine symptoms, such that the migraineur might be better off not calling in the first place. In addition, when the subject is undergoing a migraine attack, it may be physically difficult for the subject to operate the device to access digital therapeutic treatment, let alone contact a clinician to receive treatment.

### SUMMARY

To address these and other technical challenges, a service in the present disclosure is a decision support tool or digital support tool (DST) to provide timely recommendations for activities to reduce the number of migraines that a user experiences or a user's difficulty in dealing with migraines. The service may utilize user states to select and present messages to provide digital therapeutics for the given user. The service may select which activity, among multiple activities, to send to a user. The activities may contain different messages to be presented to the user, such as via a mobile device of the user. The objective of each different activity and its corresponding messages can be to reduce the number of migraines that a user experiences or a user's difficulty in dealing with migraines.

The service can identify an activity to be presented to the user via a device based on a state of the user. The states may include a migraine state, emotional states, and other assessed characteristics of the user. The migraine state may be an onset, a presence, or an absence of a migraine. The emotional state may be a calmness, a mental stamina, and a time availability of the user. The characteristic may be a migraine symptom, a vocal biomarker, a mood, or an engagement level of the user. The states may include a combination of the foregoing.

Using the user's state, the service may select an activity and associated message for the user. The activities may be categorized into calming activities or energizing activities. The activities may be categorized based on a threshold of time availability of the user. The activities may be categorized based on whether the user is experiencing an onset, a presence, or an absence of a migraine. The activities may be categorized based on a combination of the foregoing. The activities may provide behavioral therapies, including cognitive behavioral therapy, biofeedback, and relaxation therapy may also be helpful for treating migraine. The service may also time the delivery or presentation of the message to the user to avoid the user from having to actively access the digital therapeutics application on their device while suffering from migraines. Furthermore, as the service acquires additional data about the user, the service may be able to select activities and messages more targeted toward the specific user and their condition.

In this manner, the user can receive more timely help for their condition with ease to help alleviate the migraine, even when suffering a migraine which could otherwise inhibit the user from seeking treatment. This may reduce or eliminate barriers to the user from being able to contact their physician or healthcare provider for help as well as physically accessing their device while at risk of migraines. Further, the service can provide quick and objective advice that the user may rely on instead of their own subjective judgement, which may be impaired due to the current migraine. By selecting the messages sent to the user to address the subject's current state, the quality of human computer interactions (HCI) between the user and the device may be improved. In addition, since the messages are more related to the user's condition, unnecessary consumption of computational resources (e.g., processing and memory) of the service and the user device and the network bandwidth may be reduced, relative to sending ineffective messages. Furthermore, in the context of a digital therapeutics application, the individualized selection of such messages may result in the delivery of user-specific interventions to improve subject's adherence to the treatment. This may result in not only higher adherence to the therapeutic interventions but also potential improvements to subject's migraine attacks, as documented herein.

Aspects of the present disclosure are related to a system for dynamically providing messages. The system can include a computing system having one or more processors coupled with memory. The computing system can maintain, on a database, an identification of a set of activities to address migraines in a user at risk thereof. Each of the set of activities can have a set of messages to be presented in accordance with a rule. The computing system can receive, via a user interface, an indication identifying a migraine state of the user as having one of: an onset, a presence, or an absence of the migraine. The computing system can select, based on the migraine state of the user identified in the response, an activity from the set of activities to provide to the user. The computing system can identify, in accordance with the rule for the activity, a message from the set of messages. The computing system can cause the message identifying the activity to be presented via the user interface.

In some embodiments, the computing system can maintain a timer identifying a time elapsed since a presentation of a prior message via the user interface. The computing system can provide, responsive to the time elapsing a threshold, the user interface prompting for the indication of the migraine state of the user. In some embodiments, the computing system can provide, in accordance with a schedule, the user interface prompting for the indication of the migraine state of the user.

In some embodiments, the computing system can select, based on the migraine state of the user, the activity from the set of activities. The set of activities can include (i) a first activity having the set of messages associated with preventing the onset of the migraine, (ii) a second activity having the set of messages associated with alleviating the presence of the migraine, or (iii) a third activity having the set of messages associated with maintaining the absence of the migraine.

In some embodiments, the computing system can receive, via the user interface, a response identifying an activity performed by the user in response to presentation of the message of the activity. The computing system can update, on the database, a profile of the user to include the response identifying the activity performed by the user. In some embodiments, the computing system can identify the message from the set of messages for the activity in accordance with the rule based on at least one of (i) a selection of a second activity for a prior message, (ii) a response by the user to a presentation of the prior message, (iii) a number of activities performed by the user, or (iv) a taking of a medication by the user.

In some embodiments, the computing system can identify, in accordance with the rule, a group of messages from the set of messages to present within a time duration. In some embodiments, the computing system can select the activity from the set of activities based on at least one of (i) a number of occurrences of the migraine in the user, (ii) a duration of a prior occurrence of the migraine, or (iii) a taking of a medication by the user. In some embodiments, the computing system can provide, to an application on a user device, an instruction for presentation of the message to the user associated with the application.

In some embodiments, the computing system can identify, from the indication, a set of emotional states including a calmness, a time availability, and a mental stamina of the user. The computing system can select, based on the set of emotional states and the migraine state, the activity from the set of activities. In some implementations, the computing system can identify, from the indication, at least one characteristic of the user from a set of characteristics to be assessed. The set of characteristics can include one or more of a migraine symptom, a vocal biomarker, a mood, and an engagement level. The computing system can select, based on the at least one characteristic and the migraine state, the activity from the set of activities. In some embodiments, the user can be on a medication to address the migraine at least in partial concurrence with the activity.

Aspects of the present disclosure relate to a system for dynamically providing messages. The system can include a computing system having one or more processors coupled with memory. The computing system can maintain, on a database, an identification of a set of activities to address migraines in a user at risk thereof. Each of the set of activities can have a set of messages to be presented in accordance with a rule. The computing system can receive, via a user interface, an indication identifying at least one of a set of emotional states of the user. The computing system can select, based on the indication, an activity from the set of activities to provide to the user. The computing system can identify, in accordance with the rule for the activity, a message from the set of messages. The computing system can cause the message identifying the activity to be presented via the user interface.

In some embodiments, the computing system can identify, from the indication, the set of emotional states including a calmness, a time availability, and a mental stamina of the user. The computing system can select, based on the set of emotional states, the activity from the set of activities. In some embodiments, the computing system can identify, from the indication, at least one of the set of emotional states as the time availability identifying an amount of available time for the user to perform at least one of the set of activities. The computing system can select, based on a comparison of the amount of available time with a threshold time, the activity from the set of activities. The set of activities can include (i) a first activity associated with the amount of available time exceeding the threshold time and (ii) a second activity associated with the amount of available time not exceeding the threshold time.

In some embodiments, the computing system can identify, from the indication, a migraine state of the user as having one of: an onset, a presence, or an absence of a migraine. The computing system can select, based on the migraine state and the set of emotional states of the user, the activity from the set of activities. In some embodiments, the computing system can identify, from the indication, at least one characteristic of the user from a set of characteristics to be assessed. The set of characteristics can include one or more of a migraine symptom, a vocal biomarker, a mood, and an engagement level. The computing system can select, based on the at least one characteristic, the activity from the set of activities.

In some embodiments, the computing system can identify, from the indication, (i) at least one characteristic of the user from a set of characteristics to be assessed and (ii) a migraine state of the user. The computing system can select the activity based on a combination of the set of emotional states, the at least one characteristic, and the migraine state. In some embodiments, the computing system can identify, in accordance with the rule, a group of messages from the set of messages to present within a time duration. In some embodiments, the computing system can maintain a timer identifying a time elapsed since a presentation of a prior message via the user interface. The computing system can provide, responsive to the time elapsing a threshold, the user interface prompting for the indication of the set of emotional states of the user.

In some embodiments, the computing system can provide, in accordance with a schedule, the user interface prompting for the indication of the set of emotional states of the user. In some embodiments, the computing system can receive, via the user interface, a response identifying an activity performed by the user in response to presentation of the message of the activity. The computing system can update, on the database, the profile to include the response identifying the activity performed by the user. In some embodiments, the user can be on a medication to address the migraine at least in partial concurrence with the activity.

Aspects of the present disclosure relate to a method of reducing a number of migraine days in a user. The method can include presenting, by a computing device, to the user at risk of a migraine at a first time instance, a first message identifying a first activity of a set of activities. The first activity can be selected from the set of activities based on a first indication of a first migraine state of the user as one of a set of migraine states. The migraine states can include one or more of an onset, a presence, or an absence of the migraine. The method can include obtaining, by the computing device, a first metric associated with the user prior to the first activity. The method can include repeating, by the computing device, to the user at a set of second time instances, a presentation of a respective second message identifying a second activity of the set of activities. The second activity can be selected from the set of activities based on a second indication of a second migraine state of the user as one of the set of migraine states. The method can include obtaining, by the computing device, a second metric associated with the user after the second set of time instances. The method can include that the user shows a reduction in the number of migraine days, when the second metric is lower than the first metric from the first time instance.

In some embodiments, the set of activities can include (i) a first activity having the set of messages associated with preventing the onset of the migraine, (ii) a second activity having the set of messages associated with alleviating the presence of the migraine, or (iii) a third activity having the set of messages associated with maintaining the absence of the migraine. In some embodiments, the first metric at the first time instance can include a first pain catastrophizing scale (PCS) score at a baseline. The second metric at a last of the set of second time instances can include a second PCS. In some embodiments, the first metric at the first time instance can include a first PCS score at a baseline. The second metric can include a second PCS after at least one of the set of second time instances.

In some embodiments, the first metric at the first time instance can include a first number of monthly migraine days (MMDs) at a baseline. The second metric at a last of the set of second time instances can include a second number of MDDs. In some embodiments, the first metric at the first time instance can include a first number of MDDs at a baseline. The second metric can include a second number of MDDs after at least one of the set of second time instances. In some embodiments, the migraine can include at least one of a complicated migraine, a common migraine, a silent migraine, a hemiplegic migraine, or a retinal migraine.

In some embodiments, the user can be on a medication to address the migraine at least in partial concurrence over at least one of the first time instance or the set of second time instances. The medication can include at least one of a triptan, an ibuprofen, an ergot, or a calcitonin gene-related peptide (CGRP) inhibitor.

Aspects of the present disclosure relate to a method of reducing a number of migraine days in a user. The method can include presenting, by a computing device, to the user at risk of a migraine at a first time instance, a first message identifying a first activity of a set of activities. The first activity can be selected from the set of activities based on a first indication of a first set of emotional states for the user. The method can include obtaining, by the computing device, a first metric associated with the user prior to the first activity. The method can include repeating, by the computing device, to the user at a set of second time instances, a presentation of a respective second message identifying a second activity of the set of activities. The second activity can be selected from the set of activities based on a second indication of a second set of emotional states of the user. The method can include obtaining, by the computing device, a second metric associated with the user after the second set of time instances. The user can show a reduction in the number of migraine days when the second metric is lower than the first metric from the first time instance.

**In** some embodiments, the first set of emotional states and the second set of emotional states each can include a calmness, a mental stamina, and a time availability of the user. In some embodiments, at least one of the first emotional state of the second emotional state of the user can identify the time availability of the user. The set of activities can include (i) an activity associated with the time availability exceeding a threshold and (ii) an activity associated with time availability not exceeding the threshold.

In some embodiments, the first metric at the first time instance can include a first pain catastrophizing scale (PCS) score at a baseline. The second metric at a last of the set of second time instances can include a second PCS. In some embodiments, the first metric at the first time instance can include a first PCS score at a baseline and the second metric can include a second PCS after at least one of the set of second time instances. In some embodiments, the first metric at the first time instance can include a first number of monthly migraine days (MDDs) at a baseline. The second metric at a last of the set of second time instances can include a second number of MDDs.

In some embodiments, the first metric at the first time instance can include a first number of MDD at a baseline and the second metric can include a second number MDD after at least one of the set of second time instances. In some embodiments, the migraine can include at least one of a complicated migraine, a common migraine, a silent migraine, a hemiplegic migraine, or a retinal migraine. In some embodiments, the user is on a medication to address the migraine at least in partial concurrence over at least one of the first time instance or the set of second time instances. The medication can include at least one of a triptan, an ibuprofen, an ergot, or a calcitonin gene-related peptide (CGRP) inhibitor.

Aspects of the present disclosure relate to a method of reducing a number of migraine days in a user. The method can include presenting, by a computing device, to the user at risk of a migraine at a first time instance, a first message identifying a first activity of a set of activities. The first activity can be selected from the set of activities based on a first indication of a first migraine state and a first set of emotional states. The method can include obtaining, by the computing device, a first metric associated with the user prior to the first activity. The method can include repeating, by the computing device, to the user at a set of second time instances, a presentation of a respective second message identifying a second activity of the set of activities. The second activity can be selected from the set of activities based on a second indication of a second migraine state and a second set of emotional states. The method can include obtaining, by the computing device, a second metric associated with the user after the second set of time instances. The user can show a reduction in the number of migraine days when the second metric is lower than the first metric from the first time instance.

In some embodiments, the first migraine state and the second migraine state can include one or more of an onset, a present, or an absence of the migraine. In some embodiments, the first set of emotional states and the second set of emotional states can each include one or more of a calmness, a mental stamina, and a time availability of the user. In some embodiments, the first indication of the second indication can identify a characteristic of the user from a set of characteristics to be assessed. The set of characteristics can include one or more of a migraine symptom, a vocal biomarker, a mood, and an engagement level to be used to select the first activity or the second activity. In some embodiments, the first metric at the first time instance can include a first pain catastrophizing scale (PCS) score at a baseline and the second metric at a last of the set of second time instances can include a second PCS.

In some embodiments, the first metric at the first time instance can include a first PCS score at a baseline and the second metric can include a second PCS after at least one of the set of second time instances. In some embodiments, the first metric at the first time instance can include a first number of monthly migraine days (MDDs) at a baseline and the second metric at a last of the set of second time instances can include a second number of MDDs.

In some embodiments, the first metric at the first time instance can include a first number of MDD at a baseline and the second metric can include a second number MDD after at least one of the set of second time instances. In some embodiments, the migraine can include at least one of a complicated migraine, a silent migraine, a hemiplegic migraine, or a retinal migraine. In some embodiments, the user can be on a medication to address the migraine at least in partial concurrence over at least one of the first time instance or the set of second time instances. The medication can include at least one of a triptan, an ibuprofen, an ergot, or a calcitonin gene-related peptide (CGRP) inhibitor.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for dynamically selecting messages to provide to users at risk of migraines in accordance with an illustrative embodiment;
FIG. 2 depicts a block diagram for a process to receive an indication of a state and to select an activity based on the state in the system for dynamically selecting messages in accordance with an illustrative embodiment;
FIG. 3 depicts a block diagram for a process to provide messages for activities, to receive a response related to an activity, and to update a profile to include the response in the system for dynamically selecting messages in accordance with an illustrative embodiment;
FIGs. 4A-B each depict a method for presenting user interfaces to users at risk of migraines in accordance with an illustrative embodiment;
FIG. 5 depicts a sample system architecture for a system for presenting user interfaces to users at risk of migraines in accordance with an illustrative embodiment;
FIGs. 6A-6E depict example user interfaces to be presented by a system for presenting user interfaces to users at risk of migraines in accordance with an illustrative embodiment;
FIGs. 7A-7C depict example user interfaces for a system for presenting user interfaces to users at risk of migraines in accordance with an illustrative embodiment;
FIGs. 8A-8C depict example user interfaces for a system for presenting user interfaces to users at risk of migraines in accordance with an illustrative embodiment;
FIGs. 9A-9C depict example user interfaces of activities for a user to perform in accordance with an illustrative embodiment;
FIG. 10 is a flow diagram for presenting user interfaces to subjects with migraines in accordance with an illustrative embodiment;
FIG. 11 depicts a flow diagram of a method of reducing a number of migraine days in a user in need thereof, in accordance with an illustrative embodiment;
FIG. 12 depicts a time diagram of a single-center, exploratory, single-arm, 2-week study in late adolescents and adults with migraines
FIG. 13 depicts a time diagram of a randomized study for digital therapeutics for prevention of episodic migraines; and
FIG. 14 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for presenting user interfaces to subjects with migraines;
Section B describes examples of evaluating effectiveness of digital therapeutics on subjects at risk of migraines; and
Section C describes a network and computing environment which may be useful for practicing embodiments described herein.

### A. Systems and Methods for Dynamically Providing Messages to Users at Risk of Migraines

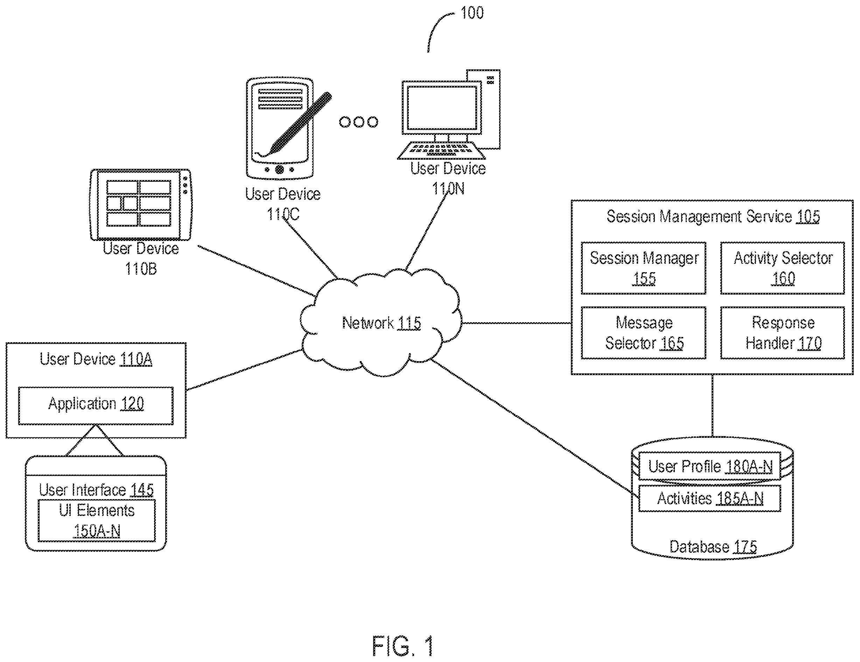

Referring now to FIG. 1, depicted is a block diagram of a system 100 for dynamically selecting messages to provide to users at risk of migraines. In an overview, the system 100 may include at least one session management service 105 and a set of user devices 110A-N (hereinafter generally referred to as user devices 110 or client devices 110), communicatively coupled with one another via at least one network 115. At least one user device 110 (e.g., the first user device 110A as depicted) may include at least one application 120. The application 120 may include or provide at least one user interface 145 with one or more user interface (UI) elements 150A-N (hereinafter generally referred to as UI elements 150). The session management service 105 may include at least one session manager 155, at least one activity selector 160, at least one message selector 165, and at least one response handler 170, , among others. The session management service 105 may include or have access to at least one database 175. The database 175 may store, maintain, or otherwise include one or more user profiles 180A-N (or user accounts; hereinafter generally referred to as user profiles 180) and one or more activities 185A-N among others. The functionality of the application 120 may be performed in part on the session management service 105.

In further detail, the session management service 105 may (sometimes herein generally referred to as a computing system or a service) be any computing device comprising one or more processors coupled with memory and software and capable of performing the various processes and tasks described herein. The session management service 105 may be in communication with the one or more user devices 110 and the database 175 via the network 115. The session management service 105 may be situated, located, or otherwise associated with at least one server group. The server group may correspond to a data center, a branch office, or a site at which one or more servers corresponding to the session management service 105 is situated. Within the session management service 105, the session manager 155 may receive, execute, or accept an indication of a user's state related to a session initiated by the user of the application 120 on respective user devices 110. The activity selector 160 may select an activity 185 to provide via the session manager to a user device 110. The message selector 165 may select, in accordance with a rule of the activity 185, a message. The response handler 170 may present the message identifying the activity 185 via the user interface 145 to the user.

The user device 110 (sometimes herein referred to as an end user computing device or client device) may be any computing device comprising one or more processors coupled with memory and software and capable of performing the various processes and tasks described herein. The user device 110 may be in communication with the session management service 105 and the database 175 via the network 115. The user device 110 may be a smartphone, other mobile phone, tablet computer, wearable computing device (e.g., smart watch, eyeglasses), or laptop computer. The user device 110 may be used to access the application 120. In some embodiments, the application 120 may be downloaded and installed on the user device 110 (e.g., via a digital distribution platform). In some embodiments, the application 120 may be a web application with resources accessible via the network 115.

The application 120 executing on the user device 110 may be a digital therapeutics application and may provide a session (sometimes referred to herein as a therapy session) to address the migraine of the user. The application 120 may be used to present messages prompting the user to perform an activity selected based on the state of the user. The state can relate to an emotional state of the user, such as a calmness of the user, a mental stamina of the user, or the time the user has available, among others. The mental stamina of the user can refer to the user's fortitude or perceived resilience for an anticipated or ongoing condition, such as a migraine. The mental stamina of the user can refer to the user's ability to concentrate on an activity. The time the user has available can refer to the time available for the user to respond, or to engage in an activity, among others. The state of the user may include, for example, a migraine state such as an onset of a migraine, an ongoing migraine, or an absence of a migraine. The state of user may include one or more characteristics to be assessed, such as migraine symptoms, a vocal biomarker, a mood, and an engagement level, among others.

The user of the application 120 may be suffering from or at risk of migraine. The migraine may be, for example: a classic migraine (sometimes herein referred to as complicated migraine) which start with an aura (e.g., flashing lights, colors, shadows, and other visual effects) prior to the head pain in the user; a common migraine without an aura and resulting in a head pain in at least one side of the head of the pain; silent migraines without pain but with the aura; a hemiplegic migraine with feelings of weakness on areas of the body such as the face, arm, or leg; retinal migraine (also herein referred to as an ocular migraine) causing a change in vision prior to head pain; among others. The user may be suffering from or at risk of headaches, such as: an icepick headache with repeated stabbing pain around eyes and temples in the user or cluster headaches with pain occurring in patterns across a period of time (e.g., day, week, or month), among others. The user may be at least partially concurrently taking medication to address the migraine, while being provided sessions through application 120. For instance, the user may be taking: a triptan (e.g., almotriptan, eletriptan, frovatriptan, naratriptan, rizatriptan, sumatriptan, or zolmitriptan), ibuprofen, aspirin, acetaminophen, dichloralphenazone-acetaminophen, an ergot (e.g., dihydroergotamine or ergotamine tartrate), or a calcitonin gene-related peptide (CGRP) inhibitor (e.g., rimegepant, ubrogepant, or zavegepant), among others.

The application 120 can include, present, or otherwise provide a user interface 145 including the one or more UI elements 150 to a user of the user device 110 in accordance with a configuration on the application 120. The UI elements 150 may correspond to visual components of the user interface 145, such as a command button, a text box, a check box, a radio button, a menu item, and a slider, among others. In some embodiments, the application 120 may be a digital therapeutics application and may provide a session (sometimes referred to herein as a therapy session) via the user interface 145 towards mitigating or reducing the effect of a migraine. For example, the application 120 can receive an instruction for presentation of a message to the user. The message can be presented textually, as an image, as a video, or other presentation to the user and can include instructions for the user to perform an activity to reduce or mitigate the effect of a migraine. The message can include instructions for the user to reduce severity of her condition. For example, the message can include instructions which aim to increase the user's calmness or mental stamina.

The database 175 may store and maintain various resources and data associated with the session management service 105 and the application 120. The database 175 may include a database management system (DBMS) to arrange and organize the data maintained thereon. The database 175 may be in communication with the session management service 105 and the one or more user devices 110 via the network 115. While running various operations, the session management service 105 and the application 120 may access the database 175 to retrieve identified data therefrom. The session management service 105 and the application 120 may also write data onto the database 175 from running such operations.

Such operations may include the maintenance of the user profile 180. The user profile 180 can include information pertaining to a condition of the user, as described herein. For example, the user profile 180 may include information related to the severity of the condition, occurrences of the condition, medications or treatments the user takes for the condition, among others. The user profile 180 may include information related to the user, such as age, sex, height, weight, location, family, allergies, profession, income, or education, among others. The user profile 180 can be updated responsive to a schedule, periodically (e.g., daily, weekly), a change in user information (e.g., input by the user via the user interface 145 or learned from the user device 110), or a clinician (e.g., a doctor or nurse) addressing the user's condition, among others. The user profile 180 can enable streamlined communications to the user by presenting a message to the user which, based on the user profile 180 at least, is most likely to aid the user in addressing her condition. This directed approach can reduce the need for multiple communications with the user, thereby reducing bandwidth and increasing the benefit of the user-computer interaction.

The user profile 180 can store and maintain information related to a user of the application 120 through user device 110. Each user profile 180 (sometimes herein referred to as a subject profile or migraineur profile) may be associated with or correspond to a respective subject or user of the application 120. The user profile 180 may identify various information about the user 210, such as information on sessions carried out by the user 210, occurrences of a condition (such as a migraine), a duration of the condition, or medications the user takes, among others. The information on a session may include various parameters of previous sessions performed by the user 210, and may initially be null.

In some embodiments, the user profile 180 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 180 may be stored and maintained in the database 175 using one or more files (e.g., extensible markup language (XML), comma-separated values (CSV) delimited text files, or a structured query language (SQL) file). The user profile 180 may be iteratively updated as the user provides responses and performs tasks of the activity 185. For example, the response handler 170 may update the user profile 180 based on a response provided by the user.

In addition, identifications of the set of activities 185 may be stored and maintained on the database 175. For example, the database 175 may maintain the identifications of activities 185 using one or more data structures or files (e.g., extensible markup language (XML), comma-separated values (CSV) delimited text files, or a structured query language (SQL) file). Each of activities 185 may define, identify, or include a message to be provided to the user via the application 120 prompting the user to perform the activity via the application 120. For example, the application 120 may receive instructions to present the message identifying the activity to be performed. The activities 185 may be to provide behavioral therapies, including cognitive behavioral therapy, biofeedback, and relaxation therapy.

In response to the presentation of the message prompting the user to perform the specified activity, the user may use the application 120 to record the performance or completion of the activity. Examples of the activities may include a breathing exercise, a mind-body exercise, or hand exercise, among others. The message can be text-based, an image, or a video, among other presentation methods. The message can be presented to the user via the user interface 145 running on the application 120. Each message may identify or include information to be presented via the user interface 145 of the application 120. For example, the information of the message may include reminders to perform a task of the session or to perform an action to mitigate a migraine. The message may be delivered periodically, such as daily, weekly, or monthly, among others. The message may be delivered according to a schedule, or responsive to reception of the indication from the user. The message may be derived from a library of pre-generated psychotherapy messages and/or a library of pre-generated engagement (remind) messages, especially as related to mitigation of migraines. The message of the activity 185 may include reminders for the subject to take medication, to practice an activity, or to perform other actions which may reduce the severity of the condition or improve the user's migraine. The message may be personalized based on the user's activity, adherence, or performance in relation to the regimen. For example, the message may be personalized based on information in the user profile 180, or based on other activities the user has participated in.

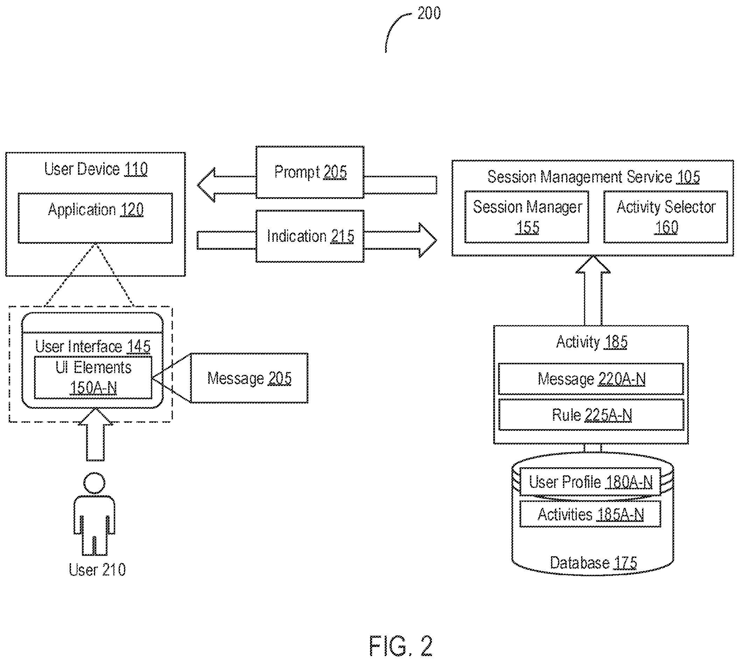

Referring now to FIG. 2, depicted is a block diagram for a process 200 to receive an indication of a state and to select an activity based on the state in the system for dynamically selecting messages . The process 200 may include or correspond to operations performed in the system 100 to present user interfaces to users with migraines. Under the process 200, the session manager 155 executing on the session management service 105 may access the database 175 to retrieve, fetch, or otherwise identify the user profile 180 for a user 210 (sometimes herein referred to as a subject, patient, person or migraineur) of the application 120 on the user device 110. The user profile 180 may identify or define information associated with the user 210, the instance of the application 120 on the user device 110, and the user device 110, among others. For example, the user profile 180 may identify that user 210 is at risk of migraines or is suffering from migraines. The user profile 180 may identify taking of medication by the user 210 to address the migraine and a frequency of migraine attacks in the user 210, among others.

With the identification of the user profile 180, the session manager 155 may determine or identify a session for the user 210 for addressing the migraine. The session may correspond to or include at least one activity to be performed by the user 210 via the application 120. The session may also have a set duration of time, ranging anywhere from between a few minutes (e.g., 3 minutes) to a few months (e.g., 3 months). The user profile 180 may include information on prior sessions, such as previous activities performed by the user 210 in response to previous messages, a response by the user 210 to the presentation of prior messages, a number of activities already performed by the user 210, and taking of medication by the user 210 to address the migraine, among others. The user profile 180 may also identify or include information on recorded migraines, such as a number of occurrences of the migraine, durations of prior occurrences, and taking of medication, among others. The user profile 180 may initially lack information about prior sessions, and may build information as the user 210 records the activities performed via the application 120.

The session manager 155 may send, provide, or otherwise transmit at least one prompt 205 for the user 210 to the user device 110 to indicate at least one state of the user 210. The prompt 205 may be to query for information related to the migraine of the user 210, such as a migraine state, one or more emotional states, or characteristics to be assessed. In some embodiments, the session manager 155 may transmit an instruction identifying the prompt 205 to the user device 110. The instruction may be to render, display, or otherwise present the prompt 205 via the one or more UI elements 150 of the user interface 145 of the application 120. The instructions may also specify a time or a condition at which to present the prompt 205 at the application 120.

In some embodiments, the session manager 155 may calculate, identify, or otherwise determine a time at which to send or present the prompt 205 via the user interface 145 of the application 120. In some embodiments, the session manager 155 may maintain a timer to count the time elapsed since the presentation of a previous prompt 205 to indicate the state of the user 210 or previous message 220 to indicate to the user 210 to perform the activity. The timer may count the time elapsed from a read receipt of the message, from a transmitted message sent from the application 120 as a part of a transfer protocol, or from the transmittal of the message from the session management service 105, among others. In some embodiments, the session manager 155 may maintain a timer to count the time elapsed since the performance of the previous activity.

Using the timer, the session manager 155 may determine whether the elapsed time exceeds a threshold. The threshold may delineate, identify, or define an amount of time at which to provide the prompt 205 for presentation. The threshold may be set or determined by the session manager 155 based on information about the user 210 as identified in the user profile 180 such as a number of occurrences of the migraine, durations of prior occurrences, and taking of medication, among others. When the elapsed time exceeds a threshold, the session manager 155 may send a prompt 205 for an indication 215 of the state of the user 210. The timing of the sending of the prompt 205 may be correlated with when the user 210 is expected to have migraine-related symptoms. Conversely, if the time counted by the timer does not exceed the threshold, the session manager 155 may refrain from sending the prompt 205 and may continue updating the time.

In some implementations, the session manager 155 may provide the prompt 205 in accordance with a schedule. The schedule may identify or specify a time at which the prompt 205 is to be presented. For example, the schedule may specify for the presentation of the prompt 205 on an hourly, daily, or weekly basis. The schedule may be defined in accordance with a prescription medicine schedule or based on the user's historical data, such as the history of migraine attacks in the user 210. When the time matches the schedule, the session manager 155 may provide the prompt 205 for display on the user interface 145 via instructions.

Upon receipt, the application 120 on the user device 110 may render, display, or otherwise present the prompt 205. The prompt 205 may be presented via the one or more UI elements 150 of the user interface 145 of the application 120 or as a push notification via the user device 110. The prompt 205 may include a text box, drop down menu, button, sliding scale, or other UI elements 150 to accept an indication 215 of state from the user 210.

In some embodiments, the application 120 can render, display, or otherwise present the prompt 205, independently of the session management service 105. The application 120 may share or have the same functionalities as the session manager 155 as discussed above. For example, the application 120 may maintain a timer to keep track of time elapsed since presentation of a previous prompt 205 or message for an activity. The application 120 may compare the elapsed time with a time limit for the message. When the elapsed time exceeds the time limit, the application 120 may determine whether to present the prompt 205. The application 120 may also use a schedule to determine when to present the prompt 205. The application 120 may present the prompt 205 for display through the user interface 145 on the user device 110. The prompt 205 may include a response mechanism, such as UI elements 150.

The prompt 205 may be to inquire the user 210 regarding the state associated with the migraine in the user 210. The state may identify or include: a migraine state, one or more emotional states, and one or more characteristics of the user 210 to be assessed. The migraine state may include one of an onset, a presence, or an absence of the migraine at the time of presentation of the prompt 205. The onset may correspond to when the user 210 expects to have a migraine soon (e.g., within a few minutes to a few hours). The presence may correspond to the user 210 concurrently undergoing a migraine attack or having experienced a migraine attack within prior time window (e.g., within a few minutes to a few hours) relative to the presentation of the prompt 205. The absence may correspond to the user 210 having no migraine related symptoms at the time of presentation of the prompt 205. For inquiring about migraine state, for example, the prompt 205 may ask the user 210 if she recently experienced a migraine, is experiencing a migraine, is anticipating a migraine, or is not experiencing any migraine symptoms.

Continuing on, the emotional states may include a calmness state, a mental stamina, and time availability, among others. The calmness may correspond to a level (or absence or presence) of tranquility indicated by the user 210 about the time of the presentation of the prompt 205. The mental stamina (or absence or presence) may correspond to a level of concentration, mental strength, or emotional wellbeing about the time of the presentation of the prompt 205. The time availability may indicate an amount of time that the user indicates to perform one or more activities at the time of presentation of the prompt 205 to address the user's condition.

For inquiring about the emotional states, the prompt 205 may further prompt for an indication of state related to the user's emotions. For example, the prompt 205 may ask the user 210 for an indication related to the user's emotional state such as the user's calmness, time availability, and a mental stamina of the user, among others. The prompt 205 may include instructions to display, via the user interface 145, push buttons for the user 210 to indicate a discrete response of state, one or more text boxes (to describe symptoms, for example), or a sliding scale to indicate a level of state, among others. For example, the user 210 may type into a text box or select from a drop-down menu, an available amount of time the user 210 has.

The prompt 205 can also prompt for additional information within the indication 215 of state. For example, the prompt 205 may ask the user for an indication 215 of the user's calmness and migraine symptoms. Likewise, the user 210 may indicate more than one state in her response. For example, the user 210 may select, on a sliding scale, a level of calmness she is experiencing, as well as select for the onset of a migraine. In some implementations, the user 210 can describe her state in text in the indication 215 and the session manager 155 can parse the text for keywords related to the user's state, such as "time", "migraine", "concentration", "feeling", etc. and can derive a state from the indication 215.

In addition, the characteristics to be assessed may identify or include a migraine symptom, a vocal biomarker, an engagement level, and mood, among others. The migraine symptoms may include, for example: pain intensity; pain location (e.g., on head, one lobe, both lobes, back or front of head); type of pain (e.g., pulsating or constant); an indication of whether the pain is worsened by a physical activity (e.g., walking or climbing); an indication of whether the physical activity was avoided due to migraine pain; indication of nausea, vomiting, sensitivity to light, sensitivity to sound, visual changes or aura, numbness; answers to migraine disability questionnaire (e.g., functional disability questionnaire for migraine disability index (MIDI)), among others.

The vocal biomarker (e.g., sample voice or speech) may include speech recording, which may be used to identify features correlated with migraines. The engagement level may identify a level of enthusiasm or proactivity of the user 210 with various activities outside those specified by the messages presented through the application 120. The mood may identify types of moods or emotions that the user 210 is concurrently facing, such as positive mood (e.g., anticipation, joy, trust, or surprise) or negative mood (e.g., fear, sadness, disgust, anger, or anxiety), among others. At least some of the information related to the characteristics may be retrieved separately from the prompt 205 or the application 120. For example, the assessment may be made by a clinician examining the user 210 with the questions (e.g., "did you take your medication today?" or "did you go on your walk?") and the assessments may be stored and maintained as part of the user profile 180. With the presentation of the prompt 205, the application 120 may monitor for at least one user interaction with the prompt 205. For example, the application 120 may monitor for the user interaction or event via event listeners or handlers in the UI elements 150 of the user interface 145 corresponding to the prompt 205. The user interactions may identify or indicate the state of the user 210 (e.g., migraine state, emotional states, or assessed characteristics, or any combination thereof) in response to the presentation of the prompt 205. When the user interaction is detected in response to the prompt 205, the application 120 may process the user interactions to determine or identify the state of the user 210 as inputted by the user 210.

Upon detection, the application 120 may generate at least one indication 215 to identify the state of the user 210. In some embodiments, the application 120 may generate the indication 215 to identify the user interactions on the prompt 205 with which to determine the state of the user 210. With the generation, the application 120 may provide, send, or otherwise transmit the indication 215 to the session management service 105. The indication 215 may identify or include the state of the user 210, such as the migraine state, the one or more emotional states, or the one or more assessed characteristics, among others. In some embodiments, the indication 215 may identify the migraine state of the user 210 as one of the onset, the presence, or the absence of the migraine. In some embodiments, the indication 215 may identify the set of emotional states, such as calmness, mental stamina, and time availability, among others. In some embodiments, the indication 215 may identify one or more of: the migraine symptom, the vocal biomarker, the engagement level, and the mood, among others. Any combination of these states may be identified in the indication 215.

The session manager 155 may retrieve, identify, or otherwise receive the indication 215 from the user device 110. Upon receipt, the session manager 155 may process or parse the indication 215 to determine, extract, or identify the state of the user 210. In some embodiments, the application 120 may determine the state of the user 210 using the user interactions on the prompt 205. The state of the user 210 may identify or include any combination of the migraine state, the one or more emotional states, or the one or more assessed characteristics, among others. With the identification, the session manager 155 may invoke the activity selector 160 using the state of the user 210.

The activity selector 160 on the session management service 105 can identify or select at least one activity 185 from the set of activities 185 based on the state of the user 210. In some embodiment, the activity selector 160 may select the activity 185 based on the user profile 180 in conjunction with the state of the user 210 identified in the indication 215. Each activity 185 may identify or include a set of messages 220A-N and a set of rules 225A-N. In each activity 185, the set of rules 225 (sometimes herein referred to as a decision support or migraine support rules) may define, identify, or otherwise specify one or more conditions for the state of the user 210 to satisfy for selection of the activity 185. The rules 225 may be configured to provide activities 185 to target the underlying causes or alleviate the symptoms of the migraine in the user 210 in a systematic, objective, and therapeutically effective manner. Each activity 185 may also identify, include, or may be associated with the set of messages 220.

Each activity 185 may be associated with the state indicated by the user 210 in the indication 215. In some implementations, the activity 185 may correspond to a migraine of the user 210. For example, the activities 185 can include routines or actions to be performed by the user 210 in response to messages 220 associated with preventing the onset of the migraine, messages 220 associated with alleviating the presence of a migraine, or messages 220 associated with maintaining the absence of the migraine. In some implementations, the activity 185 may correspond to an emotional state of the user 210. For example, the activity 185 may be selected based on the calmness of the user 210, or the mental stamina of the user 210, or a combination thereof. The activity 185 may also include, for example: a calming exercise (e.g., progressive muscle relaxation, breathing, willing hands, and napping) to induce or help the user 210 relax or get to a tranquil state; or energizing exercise (e.g., mindfulness exercise or sleeping), among others. In some implementations, the activity 185 may be selected based on the time available for the user 210, including in combination with other factors.

To select, the activity selector 160 may check or compare the state of the user 210 as identified in the indication 215 with the set of rules 225 in each activity 185. In some embodiments, the activity selector 160 may compare the user profile 180 and the state of the user 210 with the set of rules 225 in each activity 185. The information of the user profile 180 used to compare against the rules 225 may include, for example, previous activities performed by the user 210 in response to previous messages, a response by the user 210 to the presentation of prior messages, a number of activities already performed by the user 210, and taking of medication by the user 210 to address the migraine, among others. In addition, the information of the user profile 180 may include the number of occurrences of the migraine, durations of prior occurrences, and taking of medication, among others. The state of the user 210 used to compare against the rules 225 may include the migraine state, the one or more emotional states, or the assessed characteristics, among others, or any combination thereof. When the state of the user 210 or the information of the user profile 180 satisfies the specifications of the rules 225, the activity selector 160 may select the associated activity 185. Otherwise, when the state of the user 210 or the information of the user profile 180 does not satisfy the specifications of the rules 225, the activity selector 160 may not select the associated activity 185.

In some embodiments, the activity selector 160 may select the activity 185 based on the migraine state of the user 210. When the migraine state indicates the onset, the activity selector 160 may select the activity 185 including the set of messages 220 associated with preventing the onset of the migraine. When the migraine state indicates the presence, the activity selector 160 may select the activity 185 including the set of messages 220 associated with alleviating the presence of the migraine. When the migraine state indicates the absence, the activity selector 160 may select the activity 185 including the set of messages 220 associated with maintaining the absence of the migraine.

In some embodiments, the activity selector 160 may select the activity 185 based on a comparison between the time availability as identified in the state of the user 210 and a threshold time specified in at least one rule 225 of at least one activity 185. The threshold time may define an amount of available time at which to select or unselect the activity 185. At least one of the activities 185 may be associated with the amount of available time exceeding the threshold time (e.g., as specified by the rules 225). At least one of the activities 185 may be associated with the amount of available time not exceeding the threshold time (e.g., as specified by the rules 225). If the amount of available time exceeds the threshold time, the activity selector 160 may select the corresponding activity 185. If the amount of available time is not exceeding the threshold time, the activity selector 160 may refrain from selecting the corresponding activity 185.

In some embodiments, the activity selector 160 may select the activity 185 based on a combination of one or more of the migraine state, emotional states, or assessed characteristics as specified by the rule 225 in at least one of the activities 185. For instance, the set of rules 225 for at least one activity 185 may specify the onset of the migraine state, with the user 210 in a non-calm state with low stamina, high levels of anxiety, and the user taking medication. When the state of the user 210 matches the specification of the rules 225 for the activity 185, the activity selector 160 may select the activity 185. In some embodiments, the activity selector 160 may select the activity based on a combination of one or more of the migraine state, emotional states, or assessed characteristics in addition to the information in the user profile 180 as specified by the rule 225 in at least one of the activities 185. For example, the set of rules 225 for the activity 185 may specify the absence of the migraine state, with the user 210 in a calm state with high mental concentration, happy mood, and having performed at least 5 other activities. When the indication of the state and the information in the user profile 180 match, the activity selector 160 may select the activity 185.

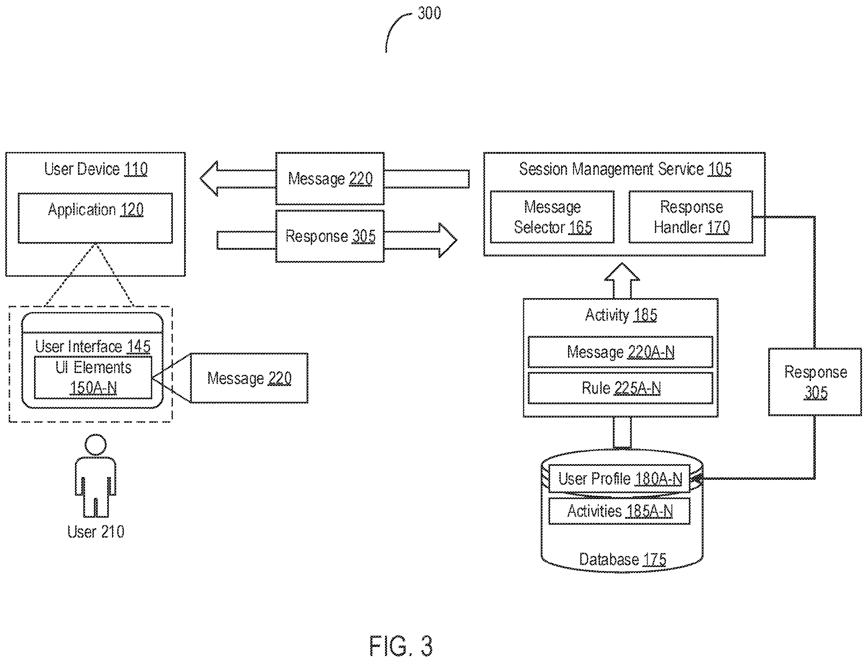

Referring now to FIG. 3, depicted is a block diagram for a process 300 to provide messages for activities, to receive a response related to an activity, and to update a profile to include the response in the system for presenting user interfaces. The process 300 may include or correspond to operations to present user interfaces to users with migraines. Under the process 300, the message selector 165 on the session management service 105 may identify or otherwise select at least one message 220 from the set of messages 220 associated with the selected activity 185 in accordance with the rule set 225. In some embodiments, the message selector 165 may select a group of messages 220 to be presented over a set time duration. Each message 220 may be a prompt for the user 210 to perform the activity 185 via the user interface 145 of the application 120 in response to presentation of the message 220. Each message 220 may provide one or more UI elements 150 when presented via the user interface 145 to allow the user 210 to input responses (e.g., indication of completion of response or answer to a questionnaire).

The set of rules 225 may also define, identify, or otherwise specify conditions for the selection of at least one of the messages 220 for the activity 185. The condition of the rules 225 may also be based on the state of the user 210, such as the migraine state, the emotional states, or the assessed characteristics. In some embodiments, the set of messages 220 may be associated with the state of the user 210, such as the migraine state, the emotional states, and the assessed characteristics, among others. For example, at least one activity 185 may include a respective set of messages 220 associated with prevention of the onset of the migraine. At least one other activity 185 may include a respective set of messages 220 associated with alleviating the presence of the migraine. At least one activity 185 may include a respective set of messages 220 associated with maintaining the absence of the migraine. In a given activity 185, the rules 225 may specify the selection of at least one message based on a combination of one or more of the migraine state, emotional states, or assessed characteristics as specified by the rule 225 in at least one of the activities 185. For example, the rules 225 for the activity 185 may specify selection of one message 220, when the state of the user 210 indicates the absence of the migraine state, with the user 210 in a calm state with high mental concentration, and happy mood.

In some embodiments, the rule 225 may specify selection of at least one message based on a time availability as identified in the state of the user 210 and a threshold time specified in at least one rule 225 of the selected activity 185. The threshold time may define an amount of available time at which to select or unselect messages 220 associated with the activity 185, and may range anywhere from seconds (e.g., 30 seconds) to hours (e.g., 3 hours). The rule 225 may specify that selection of at least one of the messages 220 when the amount of available time exceeds the threshold time (e.g., as specified by the rules 225). The rule 225 may specify that selection of at least one of the messages 220 when the amount of available time does not exceed the threshold time (e.g., as specified by the rules 225). If the amount of available time exceeds the threshold time, the message selector 165 may select the corresponding message 220. If the amount of available time does not exceed the threshold time, the message selector 165 may refrain from selecting the corresponding message 220 for the activity.

The condition of the rules 225 may be also based on the historical data in the user profile 180, such as: previous activities performed by the user 210 in response to previous messages, a response by the user 210 to the presentation of prior messages, a number of activities already performed by the user 210, and taking of medication by the user 210 to address the migraine, among others. In some embodiments, the rule 225 can specify whether at least one message 220 is to be selected based on a response by the user 210 to a presentation of a prior message. For example, the rule 225 may specify that if a previous response by the user 210 to the previously selected message 220 is incorrect, the associated message 220 is not to be selected.

Continuing on, in some implementations, the rule 225 may specify the selection of at least one message 220 is to be selected based on selection of an activity 220 for a prior message. For example, the rule 225 may specify that the message 220 for a water drinking exercise is to be selected, when the previously selected activity 185 was a physical relaxation exercise. In some embodiments, the rule 225 can specify whether at least one message 220 is to be selected based on a number of activities already performed by the user 210. For instance, the rule 225 may specify selection of a message 220 after performing five of the same type of activity 185. In some embodiments, the rule 225 may specify whether the at least one message 220 is to be selected based on a time (e.g., daytime) of day. For example, the rule 225 may prohibit messages encouraging the user 210 to go to bed when it is still daytime, prevent the selection of a message 220 which would encourage the user to take a medication outside of the prescribed medication window, or restrict a message encouraging the user to take a medication that is meant to promote sleep during the morning.

In some embodiments, the rule 225 may specify selection of at least one of the messages 220 based on a medication the user takes. For example, the rule 225 may indicate a message related to a time of medication administration, a pharmacological conflict in medications, or a reminder to take a medication, among others. The rule 225 may relate to a selection of a group of messages 220 to present within a time duration of the session. For example, the rule 225 may indicate that the messages of the group should be sent at equal intervals within the time duration of the session. Conversely, the rule 225 may indicate the messages of the group should be sent at varied frequencies within the time duration of the session. In some embodiments, the rule 225 may indicate that the messages 220 of the group are not to be sent outside of the time duration of the session. For example, if the session is active from 8AM to 4PM Monday through Friday, the rule 225 may indicate that messages related to the weekend or to nighttime are not to be selected by the message selector 165. These examples of rules 225 are meant to be illustrative and non-limiting. Any other rule not depicted herein can exist.

To select, the message selector 165 may check or compare the state of the user 210 as identified in the indication 215 with the set of rules 225 for the messages 220 of the selected activity 185. In some embodiments, the activity selector 160 may compare the user profile 180 and the state of the user 210 with the set of rules 225 in each activity 185. The information of the user profile 180 used to compare against the rules 225 may include, for example, previous activities performed by the user 210 in response to previous messages, a response by the user 210 to the presentation of prior messages, a number of activities already performed by the user 210, and taking of medication by the user 210 to address the migraine, among others. In addition, the information of the user profile 180 may include the number of occurrences of the migraine, durations of prior occurrences, and taking of medication, among others. The state of the user 210 used to compare against the rules 225 may include the migraine state, the one or more emotional states, or the assessed characteristics, among others, or any combination thereof. When the state of the user 210 or the information of the user profile 180 satisifes the specifications of the rules 225, the activity selector 160 may select the associated messages 220. Otherwise, when the state of the user 210 or the information of the user profile 180 does not satisfy the specifications of the rules 225, the activity selector 160 may not select the associated messages 220.

Upon selection, the message selector 165 may provide, send, or otherwise transmit the message 220 to the user device 110. In some embodiments, the message selector 165 may send an instruction for presentation of the message 220 via the user interface 145 for the application 120 on the user device 110. The instruction may include, for example, specification as to which UI elements 150 are to be used and may identify content to be displayed on the UI elements 150 of the user interface 145. The instructions can include the message 220. The instructions may be code, data packets, or a control to present the message 220 to the user 210 via the application 120 running on the user device 110. The instructions may include processing instructions for display of the message 220 on the application 120. The instructions may also include instructions for the user 210 to perform in relation to their session. For example, the instructions may display a message 220 instructing the user to take a medication associated with their session.

The application 120 on the user device 110 may retrieve, identify, or otherwise receive the message 220 from the session management service 105. Upon receipt, the application 120 may process or parse the message 220. The application 120 may also display, render, or otherwise present the message 220 via the user interface 145. The user interface 145 can display the message 220 as text, images, and video, among other displays. The UI elements 150 can be arranged according to the instructions or according to the application 120 to display the message 220. When presented, the message 220 may be a prompt for the user 210 to perform the selected activity 185 in a specific manner via the application 120. For instance, the message 220 may direct the user 210 to perform a stretching exercise and record a quality of experience as well as whether the exercise was completed via one or more UI elements 145 displayed on the user interface 145 as part of the message 220.

With the presentation of the message 220, the application 120 may monitor for a user interaction by the user 210 with the message 220. The message 220 can include or display prompts, buttons, text boxes, links (e.g., URLs), or other means for the user 210 to interact with or engage with the message 220. For example, the application 120 may monitor for the user interaction or event via event listeners in the UI elements 150 of the user interface 145 corresponding to the message 220 upon presentation. The user interactions may identify or indicate responses to the message 220 by the user 210 in response to the presentation. When the user interaction is detected in response to the prompt 205, the application 120 may process the user interactions to determine or identify the state of the user 210 as inputted by the user 210.

Upon detection, the application 120 may generate at least one response 305 in response to the presentation of the message 220 via the user interface 145. In some embodiments, the application 120 may generate the response 305 to identify the user interactions on the message 220. The response 305 may identify or include information pertaining to a user completion of a task, a response by the user 210, a state of the user 210, or general engagement with the message 220 or session. Upon generation, the application 120 may provide, transmit, or otherwise send the response 305 to the session management service 105.

The response handler 170 executing on the session management service 105 may retrieve, identify, or otherwise receive the response 305 from the user device 110. Upon receipt, the response handler 170 may parse the response 305 to extract or identify the state of the user 210. The response handler 170 may record or log the data from the response 305 into the database 175. The response handler 170 may store an association between the response 305 and the user profile 180. In some embodiments, the response handler 170 may store and maintain identifications of the state of the user 210 in the database 175. The response handler 170 may store an association between the response 305 and the message 220 for the user 210. For example, the response handler 170 may store information indicating that the presented message 220 elicited an improvement in the user's state from the user's state before the presentation of the message 220.

The session management service 105 may repeat the functionalities described above (e.g., processes 200 and 300) over multiple sessions. The number of sessions may be over a set number of days, weeks, or even years, or may be without a definite end point. By repeatedly selecting activities 185 based on the user states and providing messages 220 to prompt the user 210 to carry out the activities 185, the user 210 may be able to receive content to help alleviate the migraine. This may overcome the difficulties faced by the user 210 in accessing such care in a timely manner, even when suffering a migraine which could otherwise inhibit the user from seeking treatment or even physically accessing the user device 110. Furthermore, from carrying out the activities 185 prompted in the messages 220 when presented through the user interface 145 of the application 120, the quality of a human computer interactions (HCI) between the user 210 and the user device 110 may be improved.

Since the messages 220 are more related to the user's conditions (e.g., migraine state, emotional state, and assessed characteristics), the user 210 may be more likely to respond to such messages 220 when presented via the user device 110. This may reduce unnecessary consumption of computational resources (e.g., processing and memory) of the service and the user device 110 and lower the usage of the network bandwidth, relative to sending otherwise ineffectual or irrelevant messages. Furthermore, in the context of a digital therapeutics application, the individualized selection of the messages 220 using user state may result in the delivery of user-specific interventions to improve subject's adherence to the treatment. This may result in not only higher adherence to the therapeutic interventions but also lead to potential improvements to subject's migraine attacks, as documented herein in Section B (Examples).

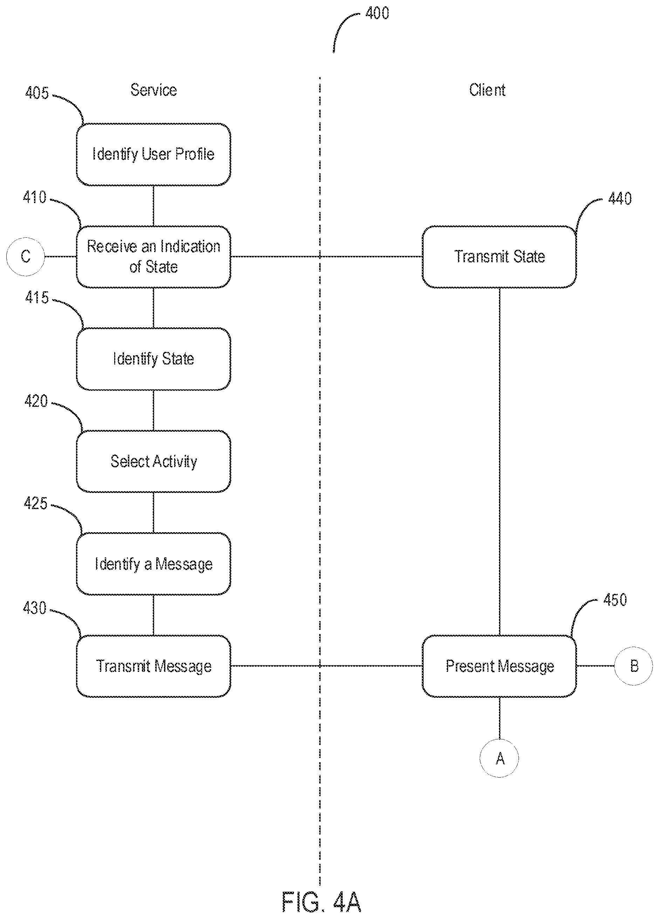

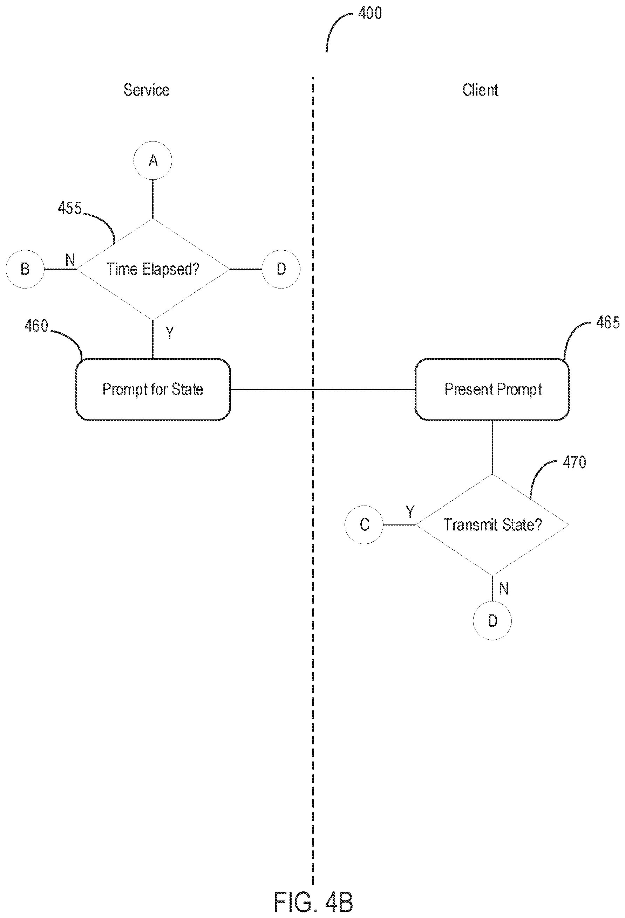

Referring now to FIGs. 4A and 4B, depicted is a method 400 for dynamically selecting messages to provide to users at risk of migraines. Each figure depicts operations performed by the client and operations performed by the service. The method 400 may be implemented or performed using any of the components detailed herein, such as the session management service 105 and the user device 110. Starting from FIG. 4A, under method 400, a service (e.g., the session management service 105) may identify a user profile (e.g., the user profile 180) (405). The client (e.g., user device 110) may transmit an indication of a state of a user (e.g., the user 210) (440). The service may receive the indication from the client (410). Upon receipt, the service may identify the state of the user (e.g., a migraine state, emotional states, or assessed characteristics) (415). The service may select an activity (e.g., the activity 185) from a set of activities based on the state (420). With the selection of the activity, the service may identify a message (e.g., messages 220) (425). The service may transmit a message to the client (430). The client may present the message to the user of the client (450).

Moving onto FIG. 4B, the service may check if a time has elapsed (455). The service may check if the time has elapsed responsive to presenting a message (450). The service may check if the time has elapsed responsive to not receiving an indication of state. If the time has not elapsed, the service may continue to present the message (450). If the time has elapsed, the service may prompt for the state (460). The client may present the prompt (465). The client may or may not transmit the indication (470). If the client transmits the indication, the process returns to (410), wherein the service receives an indication of state. If the client does not transmit the indication, the process can return to (455), wherein a determination of if the time has elapsed is made.

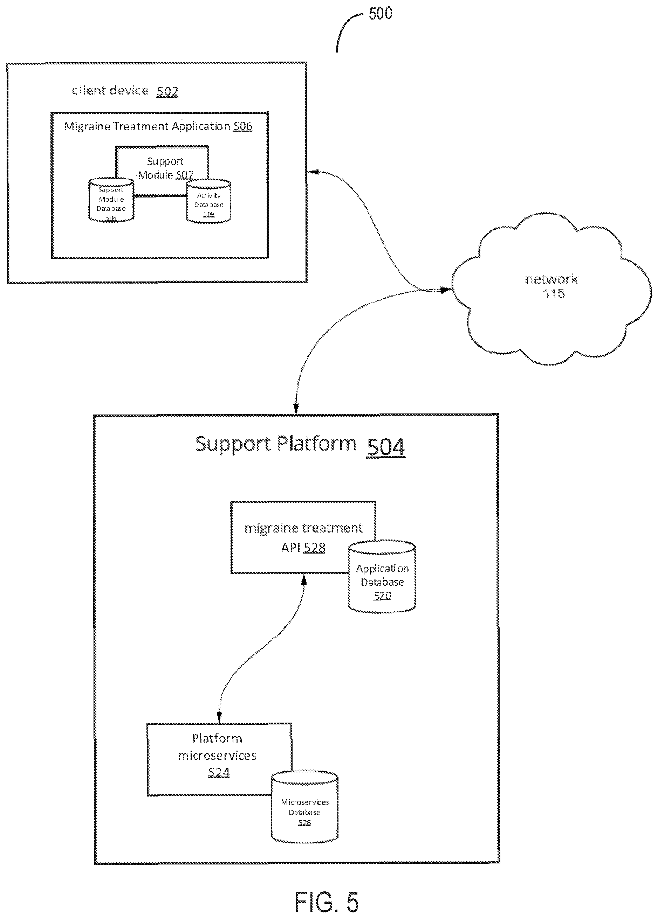

Referring now to FIG. 5, depicted is a system architecture for a system for dynamically selecting messages to provide to users at risk of migraines in accordance with an illustrative embodiment. The system can include a client device 502 (e.g., user device 110), the network 115, and a support platform 504 (e.g., the session management service 105), among others. The client device 502 can include the migraine treatment application 506 (e.g., the application 120) installed thereon. The migraine treatment application 506 may comprise a support module 507, a support module database 508, and an activity database 509, among others.

Support platform 504 may be deployed in a cloud computing environment, file server, application server, web server, proxy server, appliance, network appliance, gateway, gateway server, virtualization server, deployment server, SSL VPN server, or firewall, and may comprise a computer readable storage device, an application database 520, one or more microservices modules 524, one or more microservices databases 526, and a migraine-treatment API 528. Microservices databases 526 may comprise platform microservices such as authentication services, account services and messaging services such as SMS and email services. In some embodiments, support platform 504 may include multiple servers.

Client device 502 and support platform 504 may communicate with each other, e.g., over network 115, for instance via migraine treatment API 528. Although FIG. 5 shows a network 115 between the client devices 502 and the support platform 504, the client devices 502 and the support platform 504 may be on the same network 115. Support platform 504 may update or replace existing data in the activity database and support-module database with new or updated data. Thus, for example, platform 504 can remove a migraine-support lesson and its associated digital media content from the activity database for lessons that the migraineur has completed. Additionally or alternatively, one or more migraine-support lessons and associated digital media content can be added to the activity database for lessons that the migraineur has not yet completed. This avoids storing every migraine-support lesson and associated digital media content on client device 502 to provide more efficient memory storage.

The application can include a support feature which may also be referred to as a digital support tool or decision support tool or DST. The support feature may be used to perform a method of presenting activities to the user via the application running on the subject's mobile device. The support feature may be communicatively coupled to an activity database stored on the storage medium and to a support-module database also stored on the storage medium. A single database may comprise the activity database and the support-module database, or the activity database and the support-module database may be maintained distinct from each other.

The activity database may include activity content for (a) a group of migraine support lessons concerning science-of-migraine topics, migraine therapy topics, and migraine prevention topics, each migraine support lesson of the group of migraine support lessons comprising digital media content, (b) a group of migraine therapy activities, and (c) a group of migraine prevention activities. The activity database may be updated periodically with information received from another database, e.g., a corresponding activity database stored in a computer-readable storage medium of a remote server computer. The digital media content may comprise audio files or tracks, video, still images, animated images or videos, haptic information, textual information, and combinations thereof.

The support-module database may include a set of inquiries or prompts, such as wellness inquiries, migraine therapy inquiries and migraine prevention inquiries, and a set of decision-support rules for selecting a wellness inquiry from the plurality of inquiries, for selecting subsequent inquiries from the plurality of inquiries following a response by the user to a preceding inquiry, and for selecting the activity content or activity. The wellness inquiry may be selected from the set of inquiries based on the set of decision-support rules or based on prior inquiry responses (e.g., the subject's state responses) and is geared to having the user determine and communicate how she is currently feeling.

As such, the application permits the user to respond with a wellness response to the wellness inquiry. Wellness responses can indicate the current state of the subject and may be chosen from a group of response topics comprising (1) preventing the onset of a migraine, (2) alleviating an ongoing migraine, and (3) maintaining the absence of a migraine. The support-module database may be updated periodically with information received from another database, e.g., a corresponding support-module database stored in a computer-readable storage medium of a remote server computer.

In the context of digital therapeutic applications, the messages presented can have a causal effect on user behavior. For example, a message received from the service to the user device can cause a user to perform a task in the activity provided by the service. To facilitate ease and consistency for a user participating in an activity, the application running on the device may be used for generating and pushing prompts or inquiries to encourage the user to provide a state.

A message can include a mechanism for responding, such as a link (e.g., a uniform resource locator (URL)) within the message, a text box, or using a different platform or application also related to the application. Based on the response, further inquiries and instructions may be provided to the user based on the application's decision-support rules. Thus, if the response corresponds to the response topic of (2), alleviating an ongoing migraine, the next selected message could be seeking to reduce the severity and duration of the headache. As such, the decision-support rules require that the application provide at least one migraine therapy activity to the subject. For example, the decision-support rules may require that the application prompt as to whether the user has taken acute migraine medication since the onset of the current migraine, and if the user responds in the negative, may also require that the application advise the user to take the medication.

In this way, the application can provide quick and objective advice that the user may rely on instead of their own subjective judgement, which may be impaired due to the current migraine. The decision-support rules can include medication safety rules, however, to prevent the migraine treatment application from providing any advice to take medication when contraindicated, such as when the user has already taken the maximum amount of medication permitted. Alternatively or additionally, responsive to the state response of (2), alleviating an ongoing migraine, the decision-support rules may also require that the application provide an activity from the activity database. For example, the activity may include a pain-acceptance mindfulness exercise, such as a meditation.

If the response corresponds to a response state of (1), preventing the onset of a migraine, the service can provide a message directed towards preventing the migraine from occurring, reducing the severity of a headache that does occur, and minimizing anxiety that the user might have. As such, the decision-support rules may require that the migraine treatment application provide at least one migraine prevention activity from the activity database to the subject. For example, the decision-support rules may require that the application inquire as to whether the user has taken preventative migraine medication since becoming worried that a migraine headache might occur soon, and if the user responds in the negative, may also require that the application advise the subject, via a message presented on the mobile device, to take the medication. Again, in this way, the application provides prompt and objective advice that the user may rely on instead of their own subjective judgement, which may be impaired.

Alternatively or additionally, responsive to the response topic of (1), the application can provide a migraine prevention activity from the activity database. Migraine prevention activities include activities like drinking water and going outside for a walk and some fresh air. Other migraine prevention activities can include mindfulness exercises, such as self-compassion mindfulness exercises and curiosity mindfulness exercises.

Self-compassion exercises involve pausing to recognize when suffering is happening. Thus, pausing to focus on self-compassion helps prevent the user from focusing on her worry about the impending headache. An example of a self-compassion exercise includes thinking or saying out loud, perhaps repeatedly, "Even though it feels hopeless right now, by practicing relaxation I know I'm giving myself the best chance at feeling well. It's not easy, but this supports my long-term goal of having fewer migraines over time." Another example of a self-compassion exercise includes thinking or saying out loud, perhaps repeatedly, "Taking my medication helps my brain become less sensitive to stress, and this supports my long-term goal of having fewer migraines over time. Even though the side effect of feeling tired is hard, I'm going to stay on track with my medication schedule to support myself in being well."

Curiosity exercises also help prevent the user from focusing on the worry about the impending headache. Curiosity about migraines is an attitude that allows the user to focus on migraine symptoms without trying to change them or hope that they were something different. For example, observing bodily sensations while breathing calmly is a curiosity exercise. Further, where any uncomfortable bodily sensations are felt in the body, imagining breathing breath into that part of the body and observing how that feels permits for further observation and enables the user to be present and focused on the sensations.

Other migraine prevention activities include mind-body exercises. Mind-body exercises are physical exercises that cause beneficial emotions. Beneficial emotions, such as pleasant emotions, are useful for preventing migraines. One simple mind-body exercise is smiling. Another mind-body exercise is paced breathing. When humans are calm, their breathing is generally slower and deeper than when they are worried. Thus, subjects who practice breathing slowly and deeply can make their minds feel calm.

Another mind-body exercise is Progressive Muscle Relaxation ("PMR"). PMR is an exercise that reduces stress and anxiety in the body through slowly tensing and relaxing muscles in the body, one muscle or muscle group at a time. For example, tensing hands into a tight fists and then relaxing the hands to a neutral position might be a first step of a PMR exercise. Slowly breathing in while tensing muscles and slowly breathing out while relaxing, perhaps even slowly saying "relax" while breathing out, may also help reduce stress and anxiety.

The application, via the support module, may provide self-compassion exercises, curiosity exercises, and mind-body exercises using digital media content, such as video displayed on the display and an audio track played via the audio output. The self-compassion exercises and curiosity exercises can be provided using audio because, often, such exercises are performed with closed eyes. The mind-body exercises may be provided using video. For example, for PMR, video can help the user stay focused on which muscle or muscles should be getting tensed or relaxed.

The migraine prevention activities of drinking water and going outside for fresh air are activities that the user might know to do before she begins using the application. However, she may not know how to do other migraine prevention activities, such as practicing a migraine prevention mindfulness exercise or practicing a mind-body exercise. Thus, the decision-support rules may be structured to prevent the migraine treatment application from providing any of these exercises to the user until after she has been provided a migraine support lesson teaching about that exercise, and preferably, how to perform at least one example of such an exercise.

Specifically, the decision-support rules may require that a migraine support lesson concerning mindfulness must have been provided to the user before the migraine prevention activity of practicing a migraine prevention mindfulness exercise can be selected. Similarly, the decision-support rules may require that a migraine support lesson concerning mind-body connection must have been provided to the user before the migraine prevention activity of practicing a mind-body exercise can be selected. Thus, the only migraine prevention activities that can be selected from the activity database before the user completes any lessons are the activities of drinking water and moving to an outdoor location from an indoor location. In this manner, the application can require that the user develop knowledge about migraine science, migraine therapy, and migraine prevention before the migraine treatment application will further help the user develop her abilities with migraine therapy activities and migraine prevention activities. Developing these abilities can streamline the types of messages and activities provided to the user by the service.

If the wellness response corresponds to the response topic of (3), maintaining the absence of a migraine, then activities and messages identified by the service can correspond to maintaining that feeling or maximizing the amount of time that the user will keep that feeling. Further, times when the user is not experiencing a migraine or the onset of a migraine can provide opportunities for the service running through the application to teach the user about migraine science, migraine therapy, and migraine prevention, as well as improving the subject's abilities at practicing migraine therapy activities and migraine prevention activities. Such knowledge should help the user better understand her condition and further streamline the messaging process as a user profile of the user is updated.

Thus, upon receiving a wellness response that corresponds to the response topic of (3), maintaining the absence of a migraine, the decision-support rules could require providing at least one of the migraine support lessons concerning science-of-migraine topics, migraine therapy topics, and migraine prevention topics, at least until all of the migraine support lessons in the activity database have been provided. The decision-support rules may also require that any support lesson that introduces a migraine therapy activity or a migraine prevention activity is followed by that activity. Thus, for example, where the migraine support lesson teaches about self-compassion, the migraine support lesson should be followed by a self-compassion exercise.

By a similar vein, and as discussed above, the digital support rules might require that no migraine prevention activity be provided by the migraine treatment application until a support lesson has been provided to the user that teaches about that migraine prevention activity. Thus, for example, the digital-support rules may be set to prevent the migraine treatment application from providing any self-compassion exercises until the concept of self-compassion has been taught in at least one migraine support lesson.

As the user continues developing her abilities with the migraine prevention activities, the service can better streamline its messages provided. This can save bandwidth and computational resources, which can be crucial during an onset of a migraine for a user. The digital support rules can take advantage of any opportunity where the user feels good to teach about migraines and have the user advance her migraine prevention skills. As such, the migraine prevention activity should be at least one of practicing a migraine prevention mindfulness exercise and practicing a mind-body exercise. These exercises may be the same as some or all of the migraine prevention exercises that are provided to the user when she is anticipating a migraine, as discussed herein.

The decision-support rules may also require the application to provide a positive affirmation on the display that is automatically selected from a set of positive affirmations stored in either the activity database or the support-module database, such as "keep up the hard work" or "you are living proof that self-compassion is healing." The positive affirmations may be accompanied by an audio track or a brief animation. The application is designed to be used periodically, e.g., preferably but not necessarily at least once per day, and preferably but not necessarily at about the same time every day. Alternatively or additionally, the digital-support rules may require that the migraine treatment application automatically provide the user with the wellness inquiry at a predetermined frequency, e.g., at least once per day and at the same time every day.

If the user has not accessed the application or answered at least one wellness inquiry after a predetermined amount of time into each period or predetermined period corresponding to the predetermined frequency, such as about two to about three hours, or by a specific time of day such as noon, the digital-support rules may require that a reminder be provided to the subject, which reminder may be sent via, e.g., an SMS text message or notification. Conversely, the user might desire to use the migraine treatment application more often than the predetermined frequency. Thus, for example, if the user provides the wellness response (e.g., state) of (2), and then responds to the subsequent inquiry as to whether the user has taken acute migraine medication since the onset of the current migraine in the positive, the application will not ask about the acute medication again a second time during the period.

Notably, because the migraine treatment application operates on the subject's mobile device, or at least a mobile device that she can access easily and reliably, e.g., according to the predetermined frequency (e.g., once per day), the application provides real-time support to the subject. For example, upon receiving a response to a wellness inquiry from the subject, the migraine treatment application initiates in real-time, i.e., within at least a few milliseconds from receiving the wellness inquiry, a migraine support lesson, a migraine therapy activity, or a migraine prevention activity. Thus, for example, if the wellness response corresponds to the response topic of (2), the application may immediately provide objective advice to the user concerning migraine therapy, including objective advice about taking acute medication. Such prompt guidance cannot be achieved via in-person visits, phone calls, video conferences or even text messages between the user and healthcare providers, such as headache specialists. **In** this manner, the application is able to provide and customize activities for the user based on the user's current state, provided in responses by the user. This can create an iteratively-improving service for the user wherein overall bandwidth and data communications are minimized due to the increasing usefulness of each message.

Accordingly, after the migraine treatment application has been installed on a mobile device, the migraine treatment application may be used to perform a method for treating migraines to reduce a number of migraine-headache days that the migraineur experiences during a current time period having a number of days, e.g., twenty-eight days, as compared to a prior time period having the same number of days. During the current time period, the migraine treatment application first periodically provides the user with a wellness inquiry via the mobile device display. Thus, the migraine treatment application may provide the wellness inquiry to the migraineur upon the migraine treatment application being accessed by the user, at least once per day, and at about the same time every day. Alternatively or additionally, the migraine treatment application may automatically provide the wellness inquiry at a predetermined frequency, such as by automatically displaying the wellness inquiry on the mobile device, or by sending the wellness inquiry in a text message or through a notification. The wellness inquiry is selected from the plurality of inquiries contained in the support-module database according to the digital-support rules also contained in the support-module database.

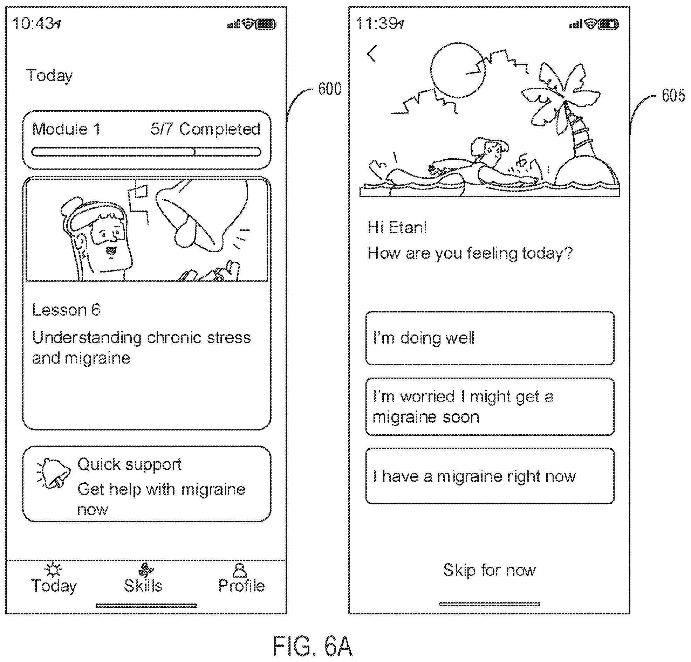

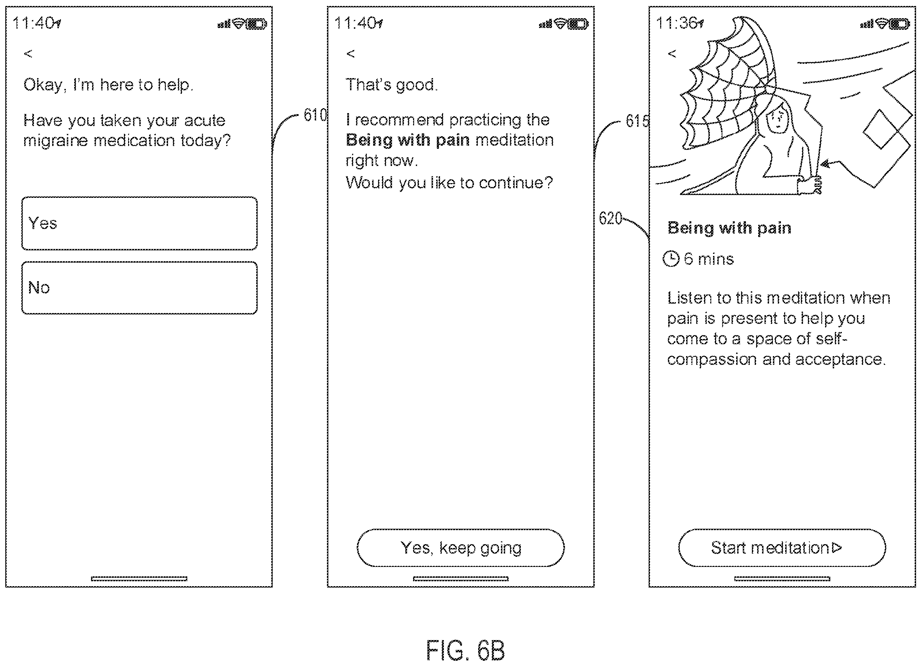

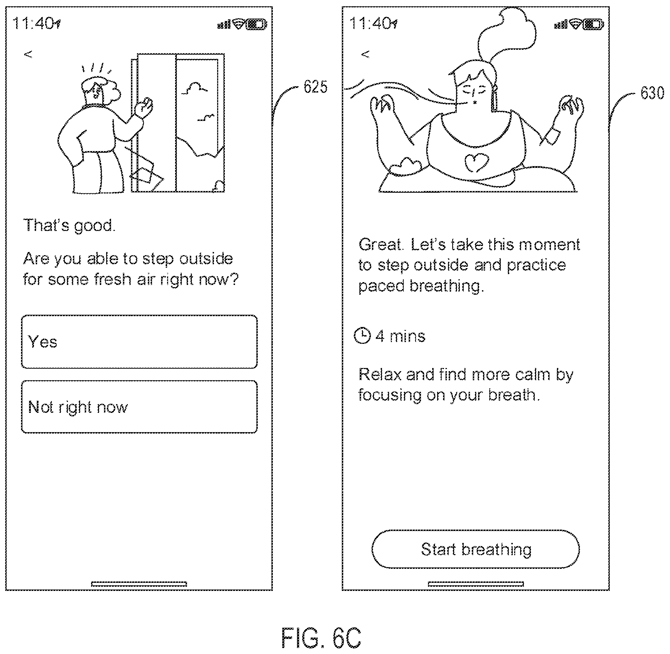

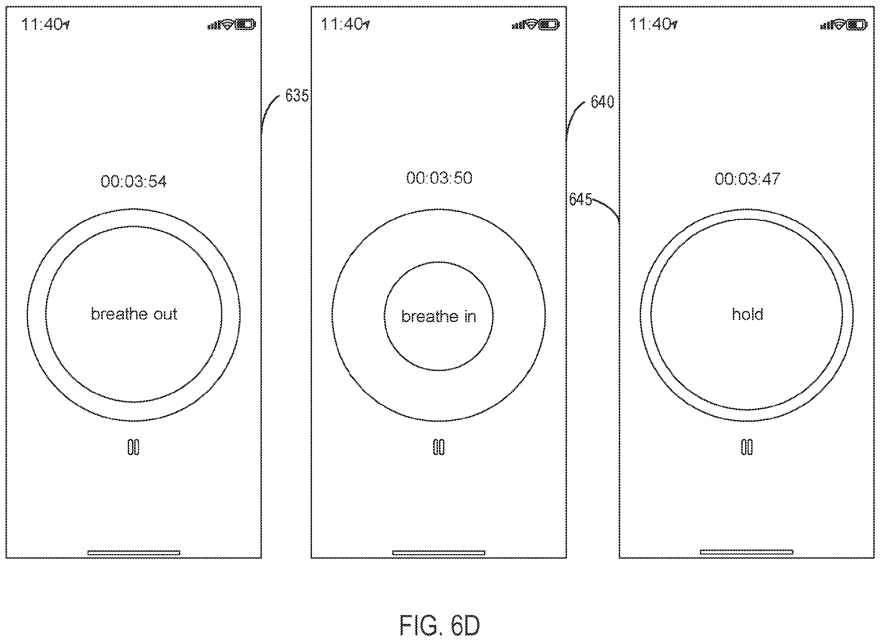

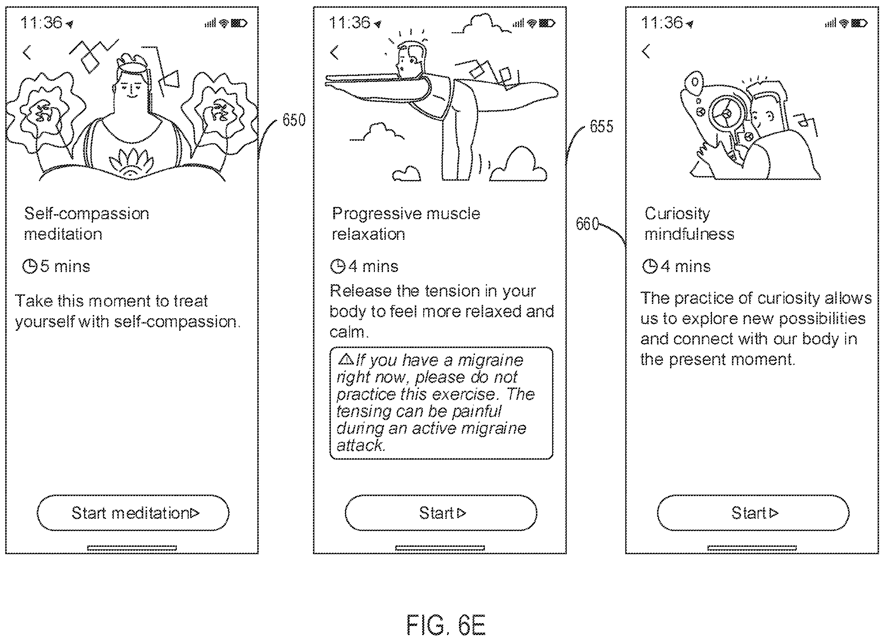

FIGs. 6A-6E depict example user interfaces (600-660) to be presented by the application 120 upon receipt of the prompt 205 or the message 220 in the system 100. The UI 600 depicts an example interface of a learning module for the user 210 to learn about migraines. The UI 605 depicts a questionnaire related to the user 210's state. The UI 610 displays questions for the user 210 related to the user's medication. In some implementations, UI 610 can display responsive to the user 210 indicating that she is experiencing or anticipates experiencing a migraine. The UI 615 displays an option for the user 210 to practice an activity to mitigate the symptoms of a migraine. The UI 620 displays a start screen for an activity. UI 625 displays a question related to an activity the user 210 may undertake. The UI 630 displays a start screen for an activity. The UIs 635-645 depict an ongoing interactive activity. Specifically, UIs 635-645 depict an example activity of breathing, with each UI 635-645 indicating a controlled breathing pattern for the user 210 to implement. UI 650 depicts an activity related to self-compassion meditation. The UI 655 depicts an activity related to progressive muscle relaxation. The UI 660 depicts an activity related to curiosity mindfulness.

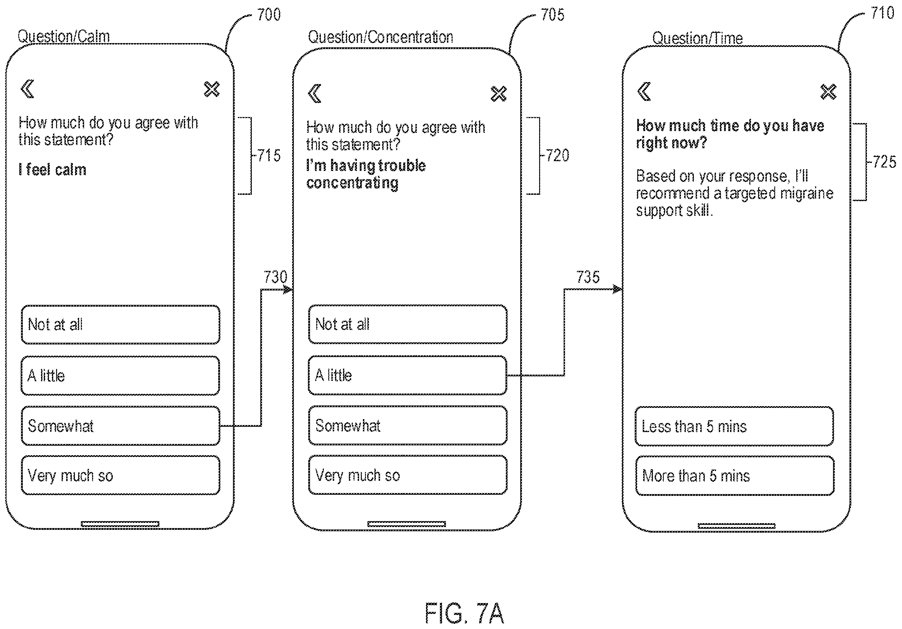

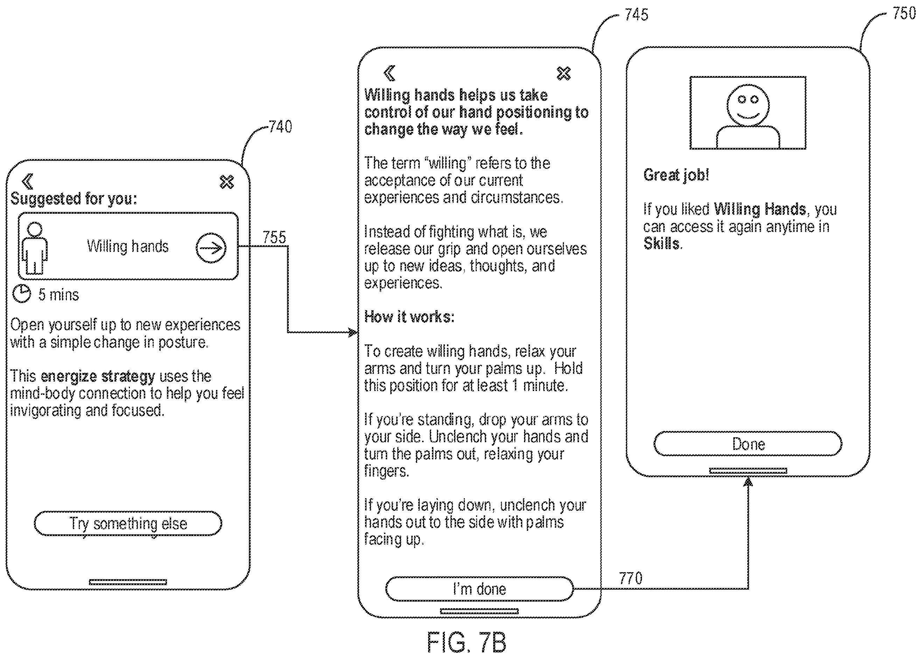

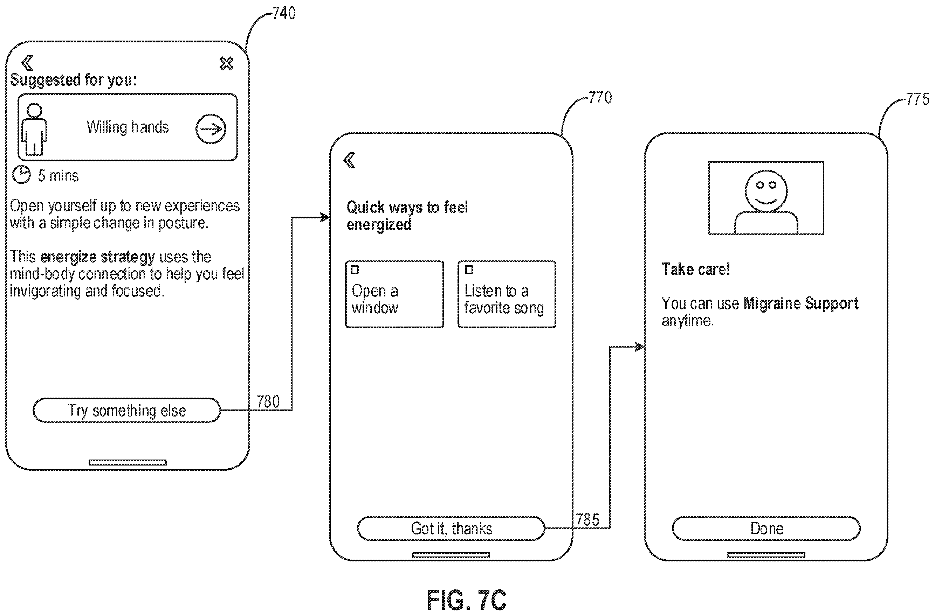

Referring now to FIGs. 7A-7C, depicted is a set of example user interfaces (700-710, 740-750, and 770-775) presented by the application 120 upon receipt of the prompt 205 or the message 220 in the system 100. The user interfaces may relate to determining a state of the user 210. These examples as depicted can relate to the prompt 205 provided by the session management service 105. In an illustrative example, the user device 110 may display via the user interface 145, an example user interface (UI) 700. The UI 700 may include a question 715 for the user. In some embodiments, the question 715 can relate to a calmness of the user 210. With the receipt of a user response 730, the user device 110 may display a second UI 705. The UI 705 may display responsive to the user selection 730. The UI 705 may pose a second question 720 to the user 210. The second question 720 may relate to a mental stamina of the user 210. The user 210 can, in some implementations, submit a second response 735. The second response 735 can prompt a third UI 710. The third UI 710 can include a third question 725. The third question 725 can relate to the amount of time a user 210 has. In some embodiments, if a user has five minutes or less, the UIs of FIGs. 7B and 7C can display.

The UI 740 can present to the user an option for an activity to undertake responsive to the user's responses to the prior questions. The user 210 can make a selection 755 of an activity. If the user chooses the selection 755, the UI 745 may display. The UI 745 can display instructions, videos, text, or other UI elements 150 to demonstrate an activity to the user 210. The user 210 can indicate completion of the activity through selection 770. With selection 770, the UI 750, indicating completion of the activity, can display. Turning now to FIG. 7C, if the user 210 selects at UI 740 selection 780, the system can present UI 770. The UI 770 can show alternate activities from those presented in UI 745. With selection 785 by the user 210, the UI 775 may display, indicating completion.

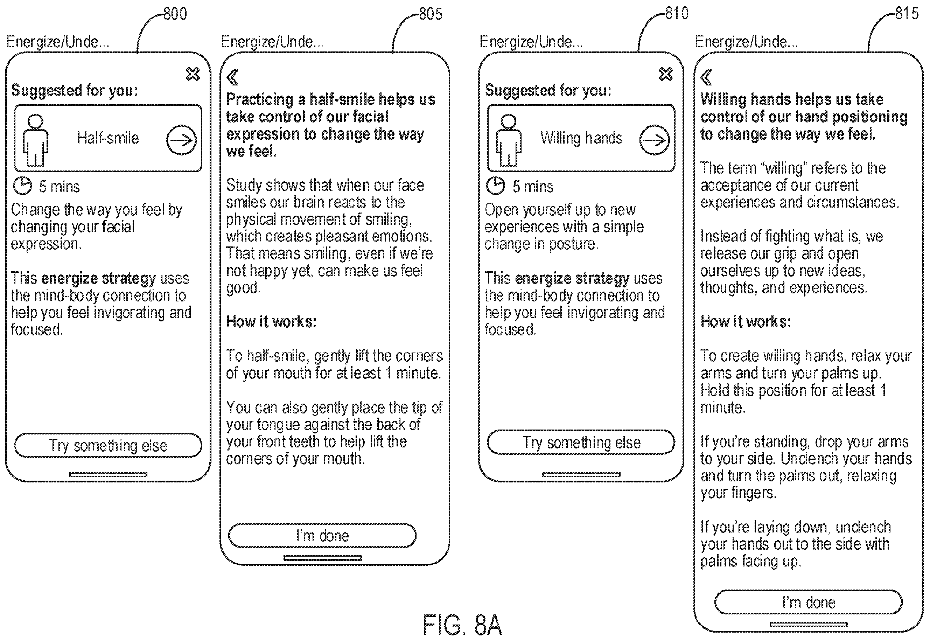

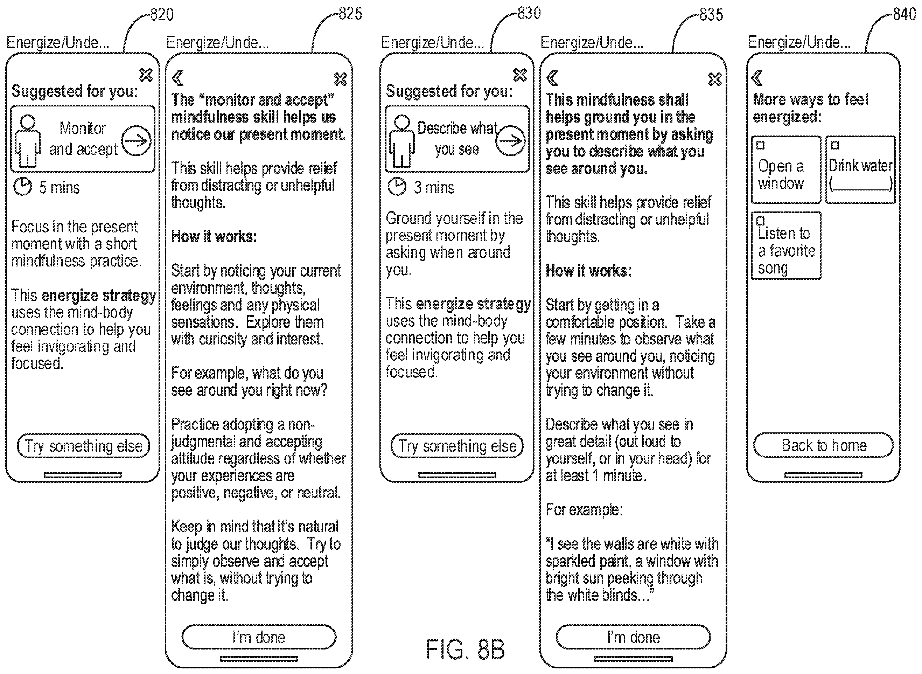

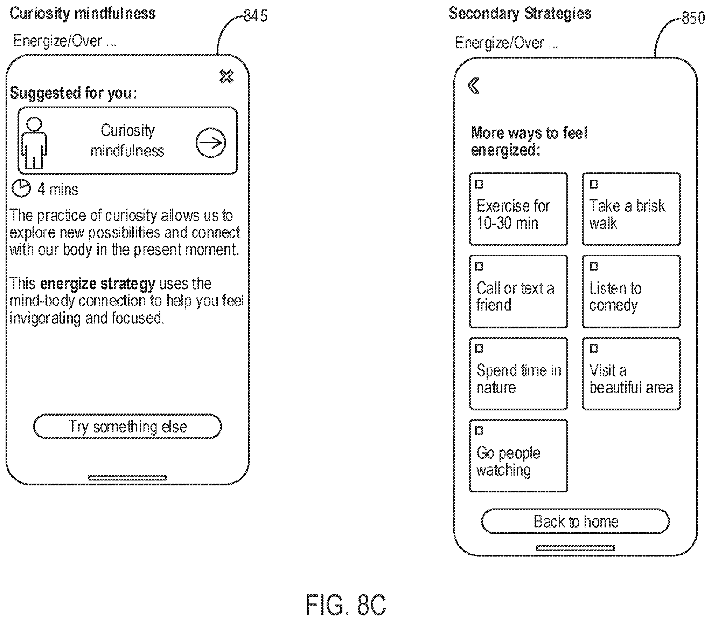

Referring now to FIGs. 8A-8C, depicted is a set of example user interfaces (800-850) presented by the application 120 upon receipt of the prompt 205 or the message 220 in the system 100. These examples as depicted can relate to the activity 185 provided by the session management service 105. In the depicted example, the user device 110 may display via the user interface 145, an example UI based on the selected activity. The UI 800 displays an activity related to a half-smile. UI 805 displays instructions associated with the activity related to a half-smile of UI 800. The UI 810 displays an activity related to willing hands. The UI 815 displays instructions associated with the activity related to willing hands of UI 810. The UI 820 displays an activity related to monitoring and accepting. The UI 825 displays instructions associated with the activity related to monitoring and accepting of UI 820. The UI 830 displays an activity related to describing what you see. The UI 835 displays instructions associated with the activity related to describing what you see of UI 830. The UI 840 displays a listing of alternate activities (e.g., secondary strategies), including opening a window and listening to a favorite song. UI 845 displays an activity related to curiosity mindfulness. The UI 850 displays a listing of alternate activities (e.g., secondary strategies), including taking a brisk walk and listening to comedy.

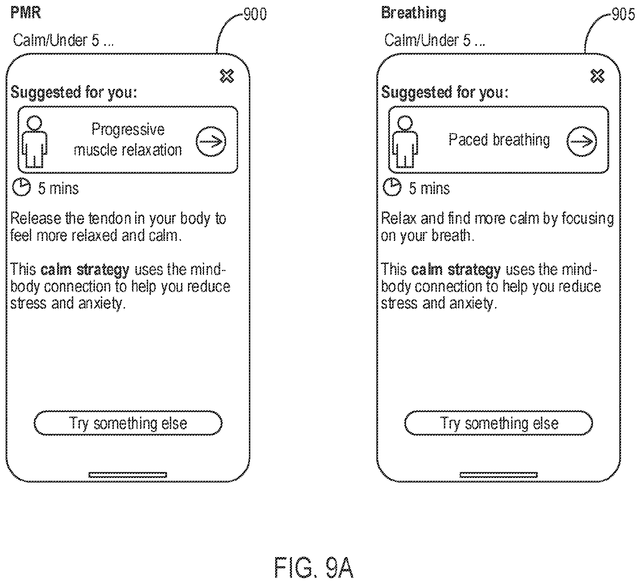

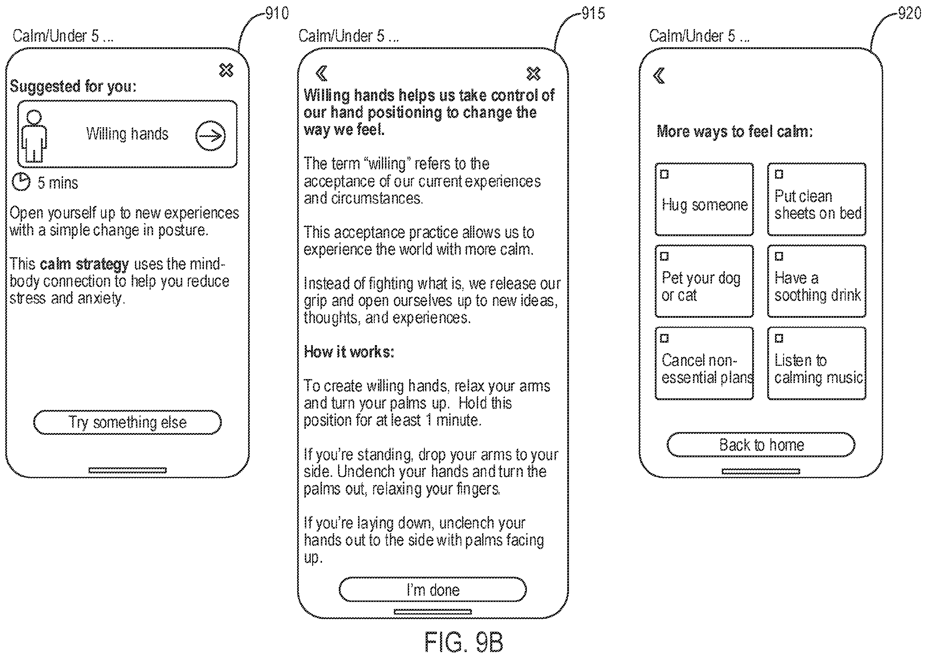

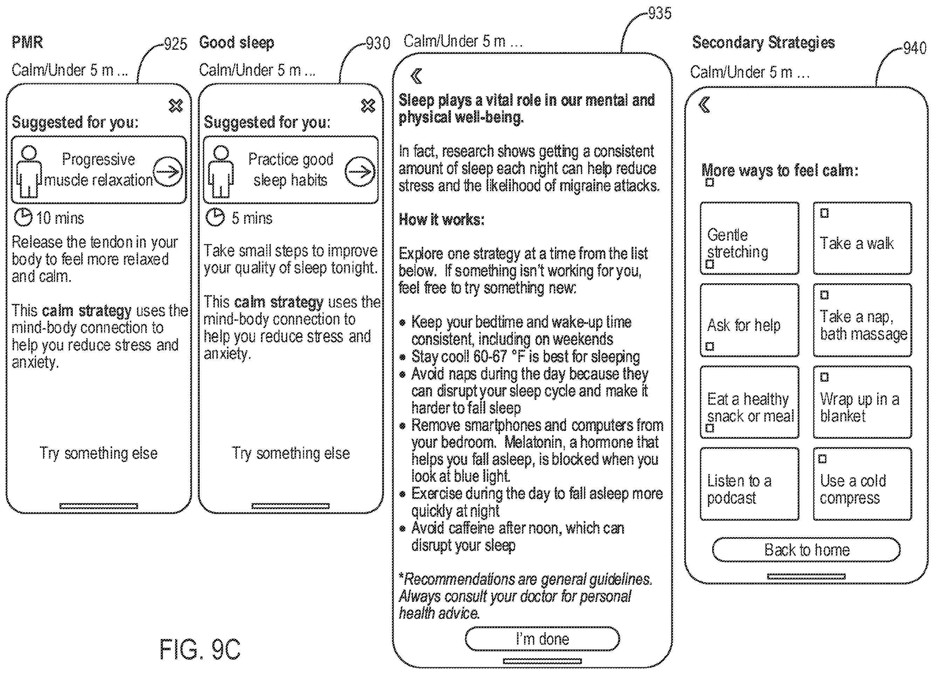

Referring now to FIGs. 9A-9C, depicted are example user interfaces (900-940) presented by the application 120 upon receipt of the prompt 205 or the message 220 in the system 100. These activities can be suggested at least based on the indication of state provided by the user 210, or the user profile 180, among others. The UI 900 depicts an activity related to progressive muscle relaxation. The UI 905 depicts an activity related to paced breathing. The UI 910 depicts an activity related to willing hands. The UI 915 depicts instructions related to the activity related to willing hands, depicted in UI 910. The UI 920 depicts a listing of example alternate activities to perform. The UI 925 depicts an activity related to progressive muscle relaxation. The UI 930 depicts an activity related to practicing good sleep habits. The UI 935 depicts instructions related to the activity related to practicing good sleep habits, depicted in UI 930. The UI 940 depicts a listing of example alternate activities to perform.

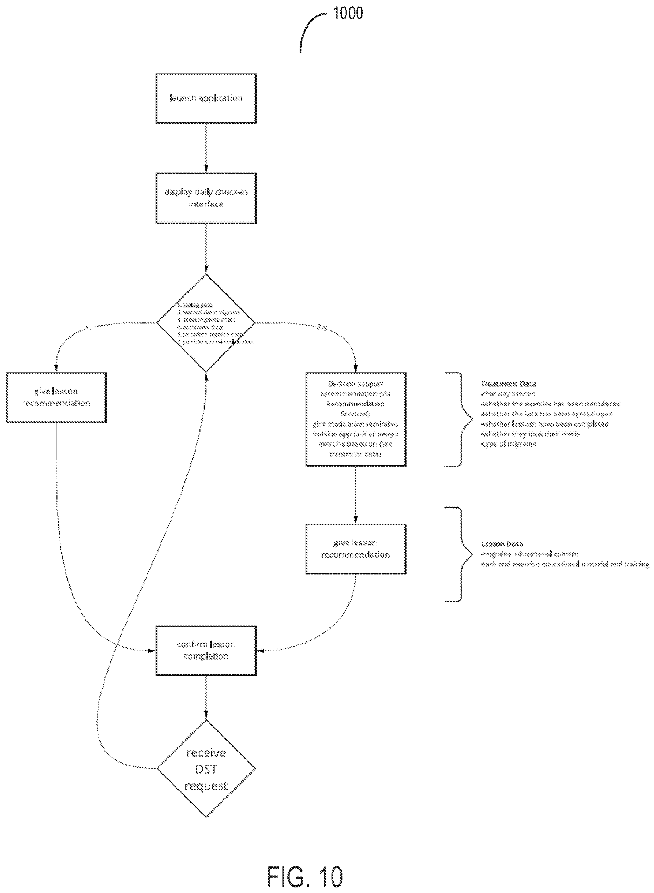

Referring now to FIG 10, depicted is a flow diagram which generally describes a method 1000 to provide lesson recommendations based on the user 210. Upon launching the migraine treatment application 506, the migraine treatment application 506 displays on the mobile-device display a daily check-in interface. It is this daily check-in interface that may include the wellness inquiry. If the migraineur 210 responds to the wellness inquiry with any response besides "feeling good," the support feature ("DST"), attempts to provide some objective advice to the migraineur 210 concerning migraine therapy or migraine prevention, and then attempts to provide the migraineur 210 with a lesson. If the migraineur 210 provides the "feeling good" response, then the support feature provides a lesson. If the lesson is completed, the support feature accounts for that so that it may provide another lesson on another day. Should the user desire to repeat this process again on the same day, the user may request this from the migraine-treatment application, and the user will again be presented with the wellness inquiry.

### B. Examples

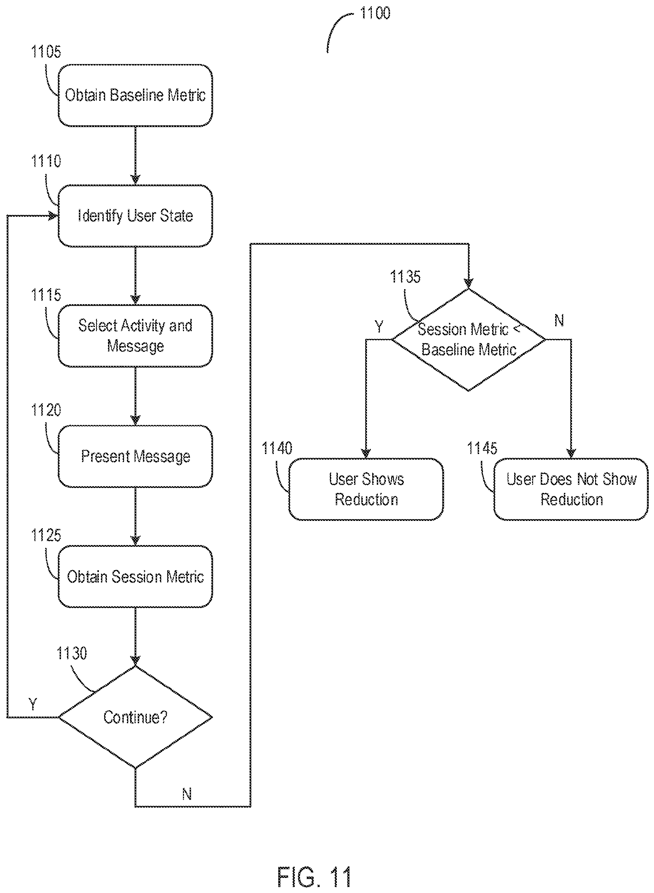

Referring now to FIG. 11, depicted is a flow diagram of a method 1100 of reducing a number of migraine days in a user in need thereof. The method 1100 may be performed by any components or actors described herein, such as the session management service 105, the user device 110, or the user 210, among others. The method 1100 may be used in conjunction with any of the functionalities or actions described herein in Sections A or B. In brief overview, the method 1100 may include obtaining a baseline metric (1105). The method 1100 may include identifying a user state (1110). The method 1100 may include selecting an activity and a message (1115). The method 1100 may include presenting the message (1120). The method 1100 may include obtaining a session metric (1125). The method may include determining whether to continue (1130). The method 1100 may include determining whether the session metric is less than the baseline metric, if the determination is to stop (1135). The method 1100 may include determining that the user shows reduction, when the session metric is less than the baseline metric (1140). The method 1100 may include determining that the user does not show reduction, when the session metric is not less than the baseline metric (1145).

In further detail, the method 1100 may include determining, measuring, or otherwise obtaining a baseline metric from a user at risk of a migraine (1105). The baseline metric may be obtained (e.g., by a clinician) prior to a user (e.g., the user 210) performing a first activity through a digital therapeutics application (e.g., the application 120) (sometimes herein referred to as CT-132 application or study app). The baseline metric may be a measure of the migraine in the user, such as a number of monthly migraine days (MDD) or a pain catastrophizing scale (PCS) score, among others. In some embodiments, the baseline metric may be Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others.

The migraine may be, for example, a complicated migraine, a common migraine, a silent migraine, a hemiplegic migraine, or a retinal migraine, among others. The user may be concurrently taking medication to address the migraine, such as: a triptan (e.g., almotriptan, eletriptan, frovatriptan, naratriptan, rizatriptan, sumatriptan, or zolmitriptan), ibuprofen, aspirin, acetaminophen, dichloralphenazone-acetaminophen, an ergot (e.g., dihydroergotamine or ergotamine tartrate), or a calcitonin gene-related peptide (CGRP) inhibitor (e.g., rimegepant, ubrogepant, or zavegepant), among others

The method 1100 may include receiving, retrieving, or otherwise identifying a user state (1110). A computing device (e.g., the session management service 105 or the user device 110) may receive an indication of at least one state of the user about a time instance. In some embodiments, the state may be one of a set of migraine states, such as an onset, a presence, or an absence of the migraine in the user. In some embodiments, the states may include a set of emotional states, such as one or more of: calmness, mental stamina, or time availability about the time instance. In some embodiments, the state may include one or more characteristics of the user to be assessed, such as a migraine symptom, a vocal biomarker, an anxiety level, an engagement level, and mood, among others. The state may include any combination of the migraine state, the set of emotional states, or the one or more characteristics.

The method 1100 may include identifying or selecting an activity (e.g., the activity 185) and a message (e.g., the message 220) for the digital therapeutics application (1115). Based on the state (e.g., migraine state, emotional states, and assessed characteristics, or any combination), the computing device may select the activity from a set of activities. Upon selection, the computing device may identify at least one message for the selected activity. The selection of the activity and the identification of the messages may be as described above in Section A.

The method 1100 may include displaying, exposing, or otherwise presenting the message to the user of the digital therapeutics application at a time instance (1120). The message may identify the selected activity to the user. The digital therapeutics application may present the message via a user interface to the user to prompt the user to perform the specified activity and to record the response to the message. The presentation of the messages and the associated functionalities may be as described above in Section A.

The method 1100 may include determining, measuring, or otherwise obtaining a session metric (1125). The session metric may be obtained (e.g., by a clinician) from the user (e.g., the user 210) after having performed at least one activity through the digital therapeutics application in response to the presentation of the message. The session metric may be a measure of the migraine in the user, such as a number of monthly migraine days (MDD) or a pain catastrophizing scale (PCS) score, among others. In some embodiments, the session metric may be Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others.

The method may include identifying or determining whether to continue providing additional messages (1130). The determination may be based on the set length (e.g., days, weeks, or years) of the trial or set number of sessions to be provided to the user. When an amount of time from the obtaining of the baseline metric or the first activity has not exceeded the set length, the determination may be to continue and to repeat from (1110). The presentations of the message identifying the selected activity may be repeated until the determination to stop. Otherwise, when the amount of time from the obtaining of the baseline metric or the first activity has exceeded the set length, the determination may be to stop.

The method 1100 may include determining whether the session metric is less than the baseline metric, if the determination is to stop (1135). In some embodiments, the determination may be performed independently of the determination whether to stop the trial. The baseline metric obtained from one time instance (e.g., prior to the first activity) may be compared against the session metric obtained from another time instance. In some embodiments, the session metric compared against may be from the last of the time instances of the repeated presentations of the messages (e.g., after determination to stop). In some embodiments, the session metric compared against may be from at least one of the time instances of the repeated presentations of the messages (e.g., independent of the determination to continue or stop).

The method 1100 may include determining that the user shows reduction, when the session metric is less than the baseline metric (1140). The reduction may be in the number of migraine days on the part of the user, when the session metric is less than the baseline metric. The type of metric may be the PCS score or the MDD, among others. In some embodiments, the reduction may be in the number of migraine days on the part of the user, when the session metric is an improvement over the baseline metric. The type of metric may be Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others. Otherwise, the method 1100 may include determining that the user does not show reduction in the number of migraine days on the part of the user, when the session metric is not less than the baseline metric (1145). In some embodiments, there may be no reduction may be in the number of migraine days on the part of the user, when the session metric is not an improvement over the baseline metric.

### Example 1 - A Single-Center, Exploratory, Single-Arm, 2-Week Study to Evaluate the Effective Establishment of a Digital Working Alliance in Late Adolescents and Adults with Migraines (Use of Migraine States for Digital Therapeutics to Address Migraines)

### Synopsis

### Study Objectives:

- To explore feasibility and acceptability of the application
- To evaluate migraine symptoms and medication use patterns when using the application
- To explore the degree of engagement with the application
- To explore compliance with daily application engagement
- To explore self-efficacy in managing a chronic condition, stress, symptoms of depression and symptoms of anxiety pre- and post 2-week use of the Application (e.g., the application 120).

### Diagnosis and main criteria for inclusion:

- Self-reported physician-diagnosed migraine
- Responded affirmatively to the question, "Have you ever been diagnosed by a physician as suffering from migraine?"
- Reported experience of at least 4 migraine days in the last 28 days
- At least 18 to 64 years of age, inclusive
- Was concurrently managing migraines with >1 prescription acute-treatment and/or preventive medications

### Test Product, Dose, Mode of Administration, and Duration of Treatment:

- The application was used on a daily basis for approximately 2 weeks by study participants on their smartphone.

### Criteria for Evaluation:

- Pain Catastrophizing Scale (PCS)
- Daily Migraine Journal
- Mobile Application Rating Scale (MARS)

### Exploratory Endpoints

- Feasibility and acceptability of the Study App, defined as:
- Degree of participant engagement with the Study App as measured by participant app use data captured in-app:
   a. Number of completed daily lessons out of total scheduled daily lessons
   b. Retention rate per day
   c. Number of times decision support tool is used per user
   d. Number of times user reports practicing a skill recommended by the Study App
- Participant feedback captured in follow-up participant qualitative interviews
- Symptom and medication use patterns via participant self-report
- Change in self-efficacy for managing a chronic disease from Baseline to Week 2, as measured by the SES6C
- Change in perceived stress from Baseline to Week 2, as measured by the PSS
- Change in symptoms of depression from Baseline to Week 2, as measured by the PHQ-8
- Change in symptoms of anxiety from Baseline to Week 2, as measured by the GAD-7

Exploratory endpoints and associated statistical analyses were performed in accordance with the SAP.

The questionnaires scores change from baseline were explored using one-sample Mest, or Wilcoxon sign-rank test. These included: SES6C; DERS-SF; PCS; PHQ-8; or GAD-7, among others.

Post-hoc exploratory analysis of MMD change over time (from baseline [BL] to end of study [EOS]) was performed using a paired t-test. Engagement metrics were reported using summary statistics (a number of non-missing observations, mean, standard deviation, median, quantiles, min and max).

Post-hoc medication adherence analysis was completed on the 34 participants who reported daily medication adherence over the previous 14 days at BL and EOS. The mean number of days on which medications were taken 14 days prior to BL was 8.1(±6) days (median 10 days), and 6.6 (±5.8) days (median 3.5 days) during the 14 days of study. Given that data was not normally distributed, medians were assessed for significance in change from BL to EOS; this difference was not statistically significant (p-value = 0.22).

Data capture methods were programmed to avoid any missing data (e.g., an answer must have been given to advance to the next assessment question). The only data that were imputed were for the number of lessons completed. This was done in instances where more advanced lessons were completed, but some of the previous lessons were missing. This situation could arise in limited circumstances of a user being offline for an extended period and prior lesson completion not being recorded (the Study App was programmed to allow access to the lessons only if the previous lessons were completed).

Study Participants:
60 participants were enrolled; there were no discontinuations.
Results:
   Engagement analysis produced the following results: The number of completed lessons ranged from 6 to 15, with mean of 13 lessons. Participants accessed the DST more than 4 times on average during the Intervention Phase and practiced nearly 4 skills on average from the DST. Participants also practiced therapeutic skills available to them outside of daily lessons an average of 3.4 times across the Intervention Phase.

A statistically significant mean difference in PCS scores between BL and EOS of -2.7 was observed (p-value = 0.012), indicative of an improvement in pain catastrophizing. PSS was deemed to be an unreliable measure (Cronbach's BL a = 0.029, EOS a = 0.17).

Post-hoc exploratory analyses demonstrated sufficient evidence to conclude that the mean number of BL monthly migraine days (MMDs) was statistically significantly different from mean MMDs at EOS after doubling EOS MMDs for comparison to BL to account for the difference in reporting periods (28 vs 14 days), tdf=51 = -2.47, p-value = 0.01686. The sample mean difference was -2.2 MMDs, with 95% CI [-3.94; -0.41].

### Safety Results:

No AEs or SAEs were reported during the study. The Study App presented minimal risks for users.

### Overall Conclusions:

The results of this exploratory study indicate participants formed a positive alliance with the Study App, adhered to daily lesson content, and engaged with available therapeutic skills and the DST. Participants also reported statistically significantly improved pain catastrophizing, and exploratory analysis suggests a pre-post reduction in MMDs. Taken together, these findings constitute an early signal of CT-132's anticipated clinical efficacy.

### CT-132 Application

CT-132 (e.g., the application 120) is a digital therapeutic designed to target a reduction in MMDs in individuals with migraine. CT-132 is a smartphone application that delivers therapeutic content and support throughout a 12-week treatment experience, presenting messages such as those depicted in FIGs. 6A to 6E.

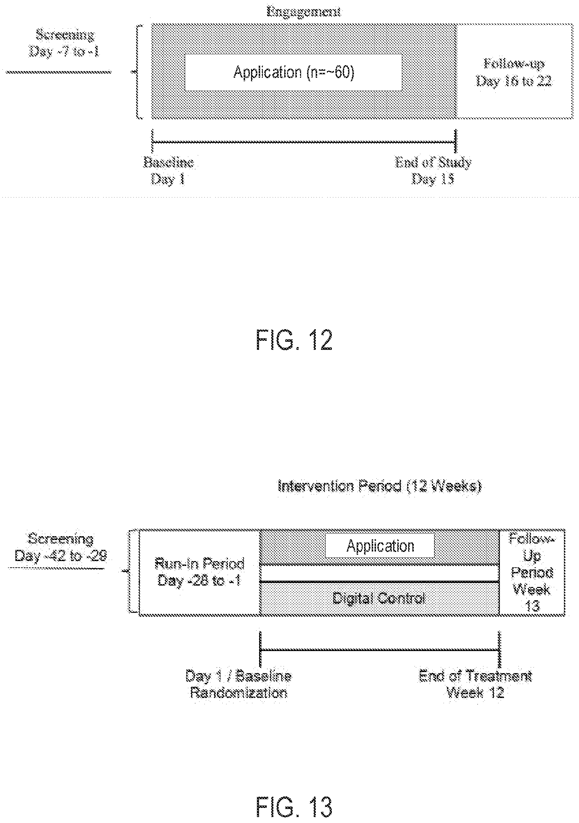

CT-132 intends to provide an interactive, software-based intervention for the prevention of migraine in late adolescents and adults aged 18-64 years. This preventive treatment will be delivered over 12 weeks, in the manner laid out in FIG. 12.

The treatment was delivered in the form of daily lessons, therapeutic skills, and a decision support tool (DST). SMS text messaging was delivered to the participant to reinforce the lessons on specific days of the 14-day Study App experience.

### Objectives and Endpoints

### Exploratory Objectives

The secondary exploratory study objectives were:
- To explore feasibility and acceptability of abbreviated treatment with the Study App
- To evaluate migraine symptoms and medication use patterns when using the Study App
- To explore the degree of engagement with the Study App
- To explore compliance with daily Study App engagement
- To explore self-efficacy in managing a chronic condition, stress, symptoms of depression, and symptoms of anxiety pre- and post-2-week use of the Study App.

This study was exploratory in nature and did not seek to evaluate specific hypotheses. Exploratory endpoints were identified. Study endpoints are listed in Table 2.

**Table 2: Study Endpoints**

| **Exploratory Endpoints** | | |
|---|---|---|
| • Feasibility and acceptability of the Study App, defined as: | | |
| | • Degree of participant engagement with the study app as measured by participant app use data captured in-app: | |
| | | • Number of completed lessons out of total scheduled lessons |
| | | • Number of daily check-ins completed* |
| | | • Retention rate per day |
| | | • Number of times decision support tool is used per user |
| | | • Number of times user reports practicing a skill recommended by the Study App |
| | • Participant feedback captured in follow-up participant qualitative interviews | |
| | • Symptom and medication use patterns via participant self-report | |
| | • Change in self-efficacy for managing a chronic disease from baseline to Week 2, as measured by the Self-Efficacy for Managing Chronic Diseases 6-item Scale (SES6C) | |
| | • Change in perceived stress from Baseline to Week 2, as measured by the Perceived Stress Scale (PSS) | |
| | • Change in symptoms of depression from Baseline to Week 2, as measured by the Patient Health Questionnaire-8 (PHQ-8) | |
| | • Change in symptoms of anxiety from Baseline to Week 2, as measured by Generalized Anxiety Disorder-7 (GAD-7) | |

| **Safety Endpoints** | | |
|---|---|---|
| • Frequency and severity of adverse events (AEs), serious AEs, and discontinuations from the study due to AEs | | |

### STUDY PARTICIPANTS

At BL, most participants (73.3%) met criteria for episodic migraine, as assessed by the ID-CM. On average participants reported severe migraine-related disability, with a mean value of 39.4 (± 35.2) on the MIDAS, and experiencing 10.3 (±8) migraines during the last 30 days at BL. Participants reported moderate levels of anxiety, with a mean value of 11.7 (±3.8), moderate levels of depression with a mean value of 13.7 (±3.9), subclinical levels of pain catastrophizing with a mean value of 17.5 (±7.9), having difficulties with emotional regulation with a mean value of 36.5 (±7.4) and self-efficacy for managing a disease had a mean value of 5.9 (±1.7). Finally, participants reported high digital literacy as assessed by the MDPQ, with the supermajority reporting they could perform the 46 listed tasks very easily.

**Table 3: Participant Baseline Scores**

| **MIDAS** | | | | |
|---|---|---|---|---|
| | Mean | | | 39.4 |
| | SD | | | ±35.2 |
| | Median | | | 32.5 |

| **ID-CM Migraine Category** | | | | |
|---|---|---|---|---|
| | | Chronic | | 16 (26.7%) |
| | | Episodic | | 44 (73.3%) |

| **ID-CM Sub-Category: Symptoms** | | | | |
|---|---|---|---|---|
| | | Criteria met | | 58 (97%) |
| | | Criteria not met | | 2 (3%) |

| **ID-CM Sub-Category: Symptoms** | | | | |
|---|---|---|---|---|
| | | Light Sensitive | | |
| | | | Never | 2 (3.3%) |
| | | | Rarely | 3 (5.0%) |
| | | | Less than half the time | 14 (23.3%) |
| | | | Half the time or more | 41 (68.3%) |
| | | Sound Sensitive | | |
| | | | Never | 0 (0.0%) |
| | | | Rarely | 4 (6.7%) |
| | | | Less than half the time | 13 (21.7%) |
| | | | Half the time or more | 43 (71.7%) |
| | | Pain Severe | | |
| | | | Never | 0 (0.0%) |
| | | | Rarely | 1 (1.7%) |
| | | | Less than half the time | 12 (20.0%) |
| | | | Half the time or more | 47 (78.3%) |
| | | Feel Sick | | |
| | | Never | 2 (3.3%) | |
| | | Rarely | 9 (15.0%) | |
| | | Less than half the time | 20 (33.3%) | |
| | | Half the time or more | 29 (48.3%) | |

| **ID-CM Sub-Category: Medication** | | | | |
|---|---|---|---|---|
| | | Criteria met | 18 (30%) | |
| | | Criteria not met | 42 (70%) | |

| **ID-CM Sub-Category: Activities** | | | | |
|---|---|---|---|---|
| | | Criteria met | 9 (15%) | |
| | | Criteria not met | 51 (85%) | |

| **ID-CM Sub-Category: Making Plans** | | | | |
|---|---|---|---|---|
| | | Criteria met | 36 (60%) | |
| | | Criteria not met | 24 (40%) | |

| **ID-CM Sub-Category: Making Plans** | | | | |
|---|---|---|---|---|
| | Interfere Plans | | | |
| | | Never | 1 (1.7%) | |
| | | Rarely | 13 (21.7%) | |
| | | Less than half the time | 28 (46.7%) | |
| | | Half the time or more | 18 (30.0%) | |
| | Worry Making Plans | | | |
| | | Never | 3 (5.0%) | |
| | | Rarely | 11 (18.3%) | |
| | | Less than half the time | 21 (35.0%) | |
| | | Half the time or more | 25 (41.7%) | |

| **ID-CM Migraine Frequency, Medication Use, Activities Sub-Scale (days)** | | | | |
|---|---|---|---|---|
| | Headache Frequency in Last 3 Months | | | |
| | | Mean | 28.9 | |
| | | SD | ±20.5 | |
| | | Median | 20 | |
| | | Min, Max | 2, 90 | |
| | Headache Frequency in Last 1 Month | | | |
| | | Mean | 10.3 | |
| | | SD | ±6.8 | |
| | | Median | 8 | |
| | | Min, Max | 3, 30 | |
| | Meds Use OTC | | | |
| | | Mean | 4.4 | |
| | | SD | ±5.2 | |
| | | Median | 3.5 | |
| | | Min, Max | 0, 30 | |

| Meds Use Prescriptions | | | | |
|---|---|---|---|---|
| | | Mean | 7.4 | |
| | | SD | ±7 | |
| | | Median | 5 | |
| | | Min, Max | 0, 30 | |
| | Miss Work / School | | | |
| | | Mean | 2.3 | |
| | | SD | ±3.4 | |
| | | Median | 1 | |
| | | Min, Max | 0, 16 | |
| | Miss Work Activities | | | |
| | | Mean | 3.8 | |
| | | SD | ±3.7 | |
| | | Median | 3 | |
| | | Min, Max | 0, 15 | |

| **SES6C** | | | | |
|---|---|---|---|---|
| | | Mean | 5.9 | |
| | | SD | ±1.7 | |
| | | Median | 5.6 | |
| | | Min, Max | 2.8, 10 | |

| **DERS-SF** | | | | |
|---|---|---|---|---|
| | | Mean | 36.5 | |
| | | SD | ±7.4 | |
| | | Median | 36.5 | |
| | | Min, Max | 28, 58 | |

| **PCS** | | | | |
|---|---|---|---|---|
| | | Mean | 17.5 | |
| | | SD | ±7.9 | |
| | | Median | 18 | |
| | | Min, Max | 2.0, 34 | |

| **PHQ-8** | | | | |
|---|---|---|---|---|
| | Mean | 13.7 | | |
| | SD | ±3.9 | | |
| | Median | 13 | | |
| | Min, Max | 8.0, 23 | | |

| **GAD-7** | | | | |
|---|---|---|---|---|
| | Mean | 11.7 | | |
| | SD | ±3.8 | | |
| | Median | 11.5 | | |
| | Min, Max | 7.0, 22 | | |

DERS-SF = Difficulties in Emotion Regulation Scale Short Form; GAD-7 = Generalized Anxiety Disorder 7-item; ID-CM = Identify Chronic Migraine; Max = maximum; Min = minimum; PCS = Pain Catastrophizing Scale; PHQ-8 = Patient Health Questionnaire-8; SD = standard deviation; SES6C = Self-Efficacy for Managing Chronic Disease 6-Item Scale

### Results and Tabulations of Individual Patient Data

### Analysis

### Endpoints

### Feasibility and Acceptability of the Study App

An endpoint was to examine participant engagement with the Study App as measured by lesson completion rate, use of the DST, and engagement with available skills content. A total of 14 daily lessons were available, and participants could engage with the DST and skills content as often as they preferred. The DST also suggested certain skills to practice in response to participant inputs; as such, participant engagement with skills content is analyzed for both skills practiced from the skills tab in the Study App and skills practiced from DST suggestion.

Most participants completed all 14 lessons, with a median of 14 lessons and an average of 13.2 lessons completed. Retention rates were high (see Table 4). No participants discontinued or were withdrawn.

**Table 4: Study App Usage**

| | **N** | **Missing** | **Mean** | **SD** | **Median** | **Min, Max** | **Q1, Q3** | **Total Count** |
|---|---|---|---|---|---|---|---|---|
| Number of completed lessons out of total | 60 | 0 | 13.2 | 1.6 | 14 | 6, 14 | 13,14 | 790 |
| Number of times decision support tool was used (DST | 42 | 0 | 4.5 | 10.5 | 2 | 1, 69 | 1, 4 | 188 |
| Number of times decision support tool was used (skills accessed via | 37 | 0 | 3.9 | 11.2 | 1 | 1, 69 | 1, 2 | 144 |
| Number of times skill recommended by the Study App was practiced (Skills done from | 60 | 0 | 3.4 | 6.9 | 2 | 1, 53 | 2, 2.25 | 204 |

| | | | | | | | | |
|---|---|---|---|---|---|---|---|---|
| DST = decision support tool; SD = standard deviation ^{a} Skills from the skills tab, as opposed to skill practice that may have been part of a daily lesson. | | | | | | | | |

The skills most completed by participants were paced breathing and self-compassion meditation (see Table 5).

**Table 5: Number of Skills Accessed via the Skills Tab**

| **Skill Type** | **Count** |
|---|---|
| **Total** | **204** |
| Curiosity mindfulness | 17 |
| Paced breathing | 58 |
| Pain-Acceptance mindfulness | 22 |
| Progressive muscle relaxation | 46 |
| Self-compassion meditation | 61 |

### Quality of Mobile Health App

The quality of the Study App was assessed using the MARS and is summarized in Table 6. This 23-item scale classifies and assesses the quality of mobile health apps from 1-5, where 1 indicates inadequate and 5 indicates excellent. Participants reported an App Quality mean score of 4.1 (±0.5) and a Subjective Quality mean of 4.7 (±0.8).

**Table 6: Mobile App Rating Scale (MARS) Outcomes**

| | **N** | **Missing** | **Mean** | **SD** | **Median** | **Min, Max** | **Q1, Q3** |
|---|---|---|---|---|---|---|---|
| **Engagement** | 60 | 0 | 3.8 | 0.7 | 3.8 | 1.8, 5 | 3.4, 4.3 |
| **Functionality** | 60 | 0 | 4.8 | 0.3 | 5.0 | 3.5, 5 | 4.7, 5.0 |
| **Aesthetics** | **60** | **0** | **4.4** | **0.6** | 4.5 | 2.3, 5 | **4.3, 5.0** |
| **Information** | **60** | **0** | 3.8 | 0.8 | 3.9 | 1.7, 5 | 3.8, 4.4 |
| **App Quality** | **60** | **0** | **4.1** | **0.5** | 4.1 | 2.3, 5 | 3.2, 4.4 |
| **App Subjective** | **60** | **0** | 3.7 | 0.8 | 4.0 | 1.3, 5 | 3.8, 4.4 |

### Daily Migraine Journal

Participants' reports from the migraine journal are summarized in Table 7.

**Table 7: Migraine Journal - Duration, Time Since Last Medication, Attack Pain**

| | **N** | **Missing** | **Mean** | **S.D.** | **Median** | **Min, Max** | **Q1, Q3** |
|---|---|---|---|---|---|---|---|
| Duration of Migraine | 237 | 1 | 7.4 | 6.4 | c | 1. 24 | 3. 9 |
| "How many hours of the last 24 hours have you had a migraine attack?" | | | | | | | |
| Time Since Last Medication (hours) | | 0 | 8.9 | 6.8 | 7.5 | 1, 24 | 3, 12.5 |
| OTC | 24 | | 11.7 | 7.3 | 11 | 1, 24 | 5, 18, |
| Triptan | 45 | | 8.9 | 2.3 | 8 | 7, 14 | 8, 8.5 |
| Rx-pain | 7 | | 6.7 | 3.6 | 7.5 | 1.5, 14 | 3.75, 8 |
| Migraine attack pain level (0-10) | 234 | 4 | 5.8 | 1.8 | 0 | 1, 10 | 5. 7 |

### Symptoms and Medication Use Patterns

Table 8 described the symptoms reported by participants during migraine attacks. The most common symptoms reported were sensitivity to light (16.3%), sensitivity to sound (14.9%), tiredness (14.0%) and nausea (13.4%).

**Table 8: Reported Symptoms During Attack**

| **Symptom Type** | **N (%)** |
|---|---|
| Symptoms Total | 966 (100) |
| Sensitivity to Light | 157 (16.3) |
| Sensitivity to Sound | 144 (14.9) |
| Sensitivity to Smell | 69 (7.1) |
| Sensitivity to Touch | 28 (2.9) |
| Nausea | 129 (13.4) |
| Vomiting | 11 (1.1) |
| Nasa Congestion | 56 (5.8) |
| Irritability | 91 (9.4) |
| Dizziness/Lightheadedness | 63 (6.5) |
| Pale Skin | 13 (1.4) |
| Feeling Very Warm | 46 (4.8) |
| Feeling Very Cold | 24 (2.5) |
| Tiredness | 135 (14.0) |

### Endpoints

The improvement in mean PCS of -2.7 was statistically significant (*p*-value = 0.012), indicating a decrease in reported pain catastrophization.

**Table 9: Endpoint Measures from Baseline to End of Study**

| Assessment | Visit | N | Missing | Mean | SD | Median | Miaxn' M | Ql, Q3 | Pie-Post, p-value |
|---|---|---|---|---|---|---|---|---|---|
| SES6C | BL | 60 | 0 | 5.9 | 1.7 | 5.6 | 2.8, 10 | 4.6, 6.8 | Wilcoxon signed rank test, *p*-value = 0.12 |
| | EOS | 60 | 0 | 6.3 | 1.7 | 6.2 | 2.3, 10 | 5.5, 7.4 | |
| DERS-SF | BL | 60 | 0 | 38.3 | 7.4 | 36.5 | 28, 58 | 42 | Wilcoxon signed rank test, *p*-value = 0.21 |
| | EOS | 60 | 0 | 39.1 | 7.6 | 38.5 | 23, 63 | 43 | |
| PCS | BL | 60 | 0 | 17.5 | 7.9 | 18 | 2.0, 34 | 11.8, 23 | *t*_{df=59} = -2.612-value = Δ*µ* = -2.7 |
| | EOS | 60 | 0 | 14.8 | 9.1 | 13.5 | 0, 38 | 8.0, 20.3 | |
| PHQ-8 | BL | 60 | 0 | 13.7 | 3.9 | 13 | 8.0, 23 | 10.8, 16 | Wilcoxon signed rank test, *p*-value = 0.64 |
| | EOS | 60 | 0 | 13.4 | 3.5 | 13 | 8.0, 22 | 11, 15.3 | |
| GAD-7 | BL | 60 | 0 | 11.7 | 3.8 | 11.5 | 7.0, 22 | 9.0, 14 | Wilcoxon signed rank test, *p*-value = 0.58 |
| | EOS | 60 | 0 | 11.3 | 3.6 | 11 | 7.0, 23 | 8.0, 13.3 | |

| | | | | | | | | | |
|---|---|---|---|---|---|---|---|---|---|
| Δ*µ* = mean difference; BL = baseline, EOS = end of study, SD = standard deviation; *t*_{df} = *t*-score with df degrees of freedom | | | | | | | | | |

### Post-hoc Analyses

### Monthly Migraine Days

Monthly migraine days were assessed as a post-hoc exploratory analysis. Note that BL MMDs were assessed by asking participants to report MMDs over the past month, while the MMD assessment period during the study was only two weeks. For this reason, EOS MMDs were multiplied by 2 to compare to BL MMDs (see Table 10). Participants with fewer than 14 daily migraine surveys were excluded from the statistical test (n = 52).

**Table 10: Summary of Monthly Migraine Days**

| **Visit** | **N** | **Missing** | **Mean** | **SD** | **Median** | **Min, Max** | **Q1, Q3** | **Pre-Post, *p*-value** |
|---|---|---|---|---|---|---|---|---|
| BL | 52 | 0 | 10.1 | 6.3 | 8 | 3, 30 | 5, 14.25 | *t*_{df=51} = -2.47, *p*-value = 0.017, 95% CI [-2.9;- |
| EOS | 52 | 8 | 7.9 | 4.5 | 8 | 0, 24 | 6, 10 | |

| | | | | | | | | |
|---|---|---|---|---|---|---|---|---|
| BL = Baseline; EOS = End of Study; *t*_{df} = *t*-score with df degrees of freedom | | | | | | | | |

Paired data with 2 observations were analyzed in this post-hoc exploratory pre-post comparison. The difference in mean in MMDs from BL to EOS was -2.2, indicating statistically significant improvement (*t*_{df=51} = -2.47, *p*-value = 0.017, 95% CI [-2.9; -0.4]).

### Preventive Medication Adherence

34 participants reported daily medication adherence over the last 14 days at BL and EOS. The mean number of days on which medications were taken 14 days prior to BL was 8.1(±6) days (median 10 days), and 6.6 (±5.8) days (median 3.5 days) during the 14 days of study.

### Statistical/Analytical Issues

Baseline MMDs were assessed by asking the number of migraine days during the previous month, while the MMD assessment period was only two weeks. For this reason, the end of study MMDs were multiplied by 2 to make the assessment comparable to the Baseline MMDs. Data on MMDs was collected in the daily migraine survey with the Yes/No response to the question, "Have you had a migraine attack in the last 24 hours?" Participants with fewer than 14 daily recorded events on this survey were excluded from the statistical test. The pre-post comparison was done only on the paired data with 2 observations.

### No adjustments for multiplicity were made for any of the statistical tests.

Cronbach's alpha was used to explore the reliability of assessments used during this study. Assessments with α ≥ 0.6 were considered reliable. PSS assessment was deemed unreliable based on the Cronbach's alpha result (BL *α* = 0.029, EOS *α =* 0.17), thus results are not reported.

### Conclusions

Participants also engaged consistently with the Study App throughout the Intervention Phase. The median number of completed lessons (14 out of 14 possible) indicated that most participants completed every available lesson, with an average completion of more than 13 lessons. Promising utilization of Study App features was also observed, with participants who accessed the DST doing so more than four times on average during the Intervention Phase, and those who practiced skills from DST practicing nearly four skills on average. Participants also practiced therapeutic skills available to them outside of daily lessons an average of 3.4 times during the Intervention Phase.

Trends toward improvement were observed in other measures in exploratory analyses. A statistically significant reduction in pain catastrophizing observed when comparing BL and EOS, with a mean decrease in PCS scores of -2.7 with 95% CI [-4.8; -0.6] (*p*-value = 0.012). Post-hoc analyses revealed a statistically significant difference in the mean MMDs from BL to EOS of -2.2 (*p*-value = 0.017) was also observed, indicating that participants experienced a reduction in the number of migraine days per month. However, this pre-post comparison relied upon EOS MMDs that were multiplied by 2 for comparison to BL in order to account for the difference in reporting periods (28 vs 14 days). Additional post-hoc analysis failed to detect improvement in preventive medication adherence.

These results indicate participants formed a positive alliance with the Study App, adhered to daily lesson content, and engaged with available therapeutic skills and the DST. Participants also reported significantly improved pain catastrophizing, and exploratory analysis suggests a pre-post reduction in MMDs. Taken together, these findings serve as an early signal of CT-132's anticipated clinical efficacy.

### SAFETY EVALUATION

Adverse events (AEs) and serious adverse events (SAEs) were collected from the signing of the informed consent form through the end of follow-up period. No AEs were reported during this study. The Study App presents minimal risk to users.

### Example 2 - Randomized Study of Digital Therapeutics for Prevention of Episodic Migraines

### Synopsis

Indication: CT-132 is an investigational prescription digital therapeutic (PDT) indicated for the preventive treatment of episodic migraine in late adolescents and adults 18 years of age or older.

Study Rationale: CT-132 is a PDT being developed by Click Therapeutics. CT-132 is a mobile application that provides an interactive, software-based intervention for the preventive treatment of episodic migraine in late adolescents and adults. Example messages to be delivered to users are shown in FIGs. 7A to 9C.

The purpose of this study is to evaluate the efficacy and safety of CT-132 relative to a Digital Control in a randomized controlled trial with an adequate sample size of participants diagnosed with episodic migraine. If demonstrated to be efficacious, CT-132 would offer an important addition to currently available treatment options.

Objective: To evaluate the efficacy and safety of CT-132 in reducing the number of monthly migraine days (MMDs), compared with a Digital Control, among late adolescents and adults with episodic migraine.

Criteria for Evaluation: All efficacy endpoints are evaluated as a comparison between the treatment group and the control group.

A participant's baseline MMD and MHD will be the total number of migraine days recorded during the 28-day run-in period. The Week 12 MMD and MHD will be the total number of migraine days recorded over the previous 28 days (Weeks 9-12).

### Primary Efficacy Endpoint

- Change from baseline in the number of MMD at Week 12

### Secondary Efficacy Endpoints

- Proportion of participants who have at least a 50% reduction from baseline in the mean number of MMD at Week 12
- Change from baseline in the number of MMD recorded over the previous 28 days at Week 4 and Week 8
- Reduction from baseline in the mean number of MMD over 12 weeks
- Change in the average severity of headache from the run-in period to Weeks 9-12
- Change from baseline in the Migraine-Specific Quality-of-Life Questionnaire v2.1 (MSQ) at Week 4, Week 8, and Week 12
- Change from baseline in the Migraine Disability Assessment (MIDAS) at Week 4, Week 8, and Week 12
- Change in the use (frequency, dose, and type) of acute migraine medications from the run-in period to Weeks 9-12

### Exploratory Endpoints

- Key engagement metrics
- Participant-reported information to include, for example, data:
   ∘ To evaluate health care utilization via the Custom Healthcare Utilization Method (CHUM), by change from baseline at Week 4, Week 8, and Week 12
   ∘ To evaluate anxiety and depression via the GAD-7 and PHQ-8, respectively, by change from baseline at Week 4, Week 8, and Week 12
   ∘ To evaluate frequency of acute medication use at Week 4 and Week 8 in comparison to the run-in period
- Change from baseline in adherence to preventive medication as evaluated by the Voils DOSE-Nonadherence measure at Week 12
- Monthly headache days (MHDs)
- Change from baseline in the number of MHDs at Week 12
- Proportion of participants who have at least a 50% reduction from baseline in the mean number of MHDs at Week 12
- Change from baseline in the number of MHDs recorded over the previous 28 days at Week 4 and Week 8
- Reduction from baseline in the mean number of MHDs over 12 weeks

### Safety Endpoints

- Frequency and severity of AEs, serious AEs, and discontinuation from the study due to AEs
- Frequency and severity of AEs related to the worsening of migraine

Study Design: This is a randomized double-blind, digital-controlled, parallelgroup, virtual study to assess the efficacy and safety of CT-132 in late adolescents and adults for the prevention of migraine in comparison to a Digital Control.

The study consists of an up to 14-day screening period, a 28-day run-in period, a 12-week double-blind intervention period, and an up to 1 week follow-up period for safety assessments, as shown in FIG. 13.

### Screening Period (Day -42 to -29)

All participants will enter a screening period of up to 14 days to determine eligibility. Eligible participants must report having had at least four but no more than fourteen migraine days per month in the three months prior to the Screening Visit (a month is defined as 28 days).

Prior to entering the screening period, participants will be asked to download the ObvioHealth App (separate from the Study App) in order to provide consent to the trial and complete trial assessments, including self-administered scales.

### Run-In Period (Day -28 to -1)

After completing the study onboarding activities, eligible participants will be contacted to schedule a remote video call to verify their identity and their migraine medication prescriptions. Once reviewed and verified, participants will be introduced to the Study App by downloading and installing the application onto their personal iPhone or Android smartphone. Only the eDiary component of the Study App will be activated.

Participants will then enter a 28-day run-in period during which they will enter daily headache information within the Study App to establish their baseline MMDs and determine Study App adherence and performance. Adherence to the onboarding requirements will be assessed by the investigator prior to randomization at the baseline visit.

### Baseline Virtual Visit (Day 1)

After completing the 28-day run-in period, the participant will be contacted for a Baseline Visit to review and confirm eligibility. Participants will be considered eligible for study entry based on all of the following criteria:
- Continuing to meet all other inclusion and no exclusion criteria, based on investigator assessment.
- Understanding of and adherence to the onboarding requirements and Study App (at 80% or greater adherence).
- Reporting within the Study App between 4-14 migraine days (inclusive) during the 28-day run-in period.
- Agreeing to complete the 12-week study intervention in good faith at the time of baseline virtual visit.

Eligible participants will then be randomly assigned in a 1:1 ratio (CT-132: Digital Control) and the assigned full therapeutic app will be activated. Assessments and activities during this period will be performed remotely by telemedicine technology visits according to the SoA (Section 1.2).

### Double-Blind Intervention Period (12 Weeks)

During the 12-week intervention period, the Study App will deliver either CT-132 or the Digital Control. Participants may use their prescribed medication to treat episodic migraine (including changes in medication or dosage) as needed while continuing to use the Study App on their regular schedule. Assessments and activities during this period will be performed remotely according to the Schedule of Activities and Assessments (Section 1.2).

### Follow-Up Period (Up to 1 week)

Within one week of completion of the 12-week intervention period or withdrawal from the study, participants will complete the follow-up activities according to the Schedule of Activities and Assessments (Section 1.2).

Planned Number of Participants: Approximately 558 participants will be randomized in this study.

Study Centers: This will be a fully virtual study. Participants living in the United States will be enrolled and followed remotely through telemedicine visits.

### Study Entry Criteria: Inclusion Criteria

A participant will be eligible for entry into the study if all of the following criteria are met:
1. Willing and able to provide written informed consent to participate in the study, attend study visits, and comply with study-related requirements and assessments.
2. Lives in the United States.
3. Adult or late adolescent, 18 years of age or older at the time of informed consent.
4. Fluent in written and spoken English, confirmed by ability to read and understand the informed consent form.
5. The following will be physician-reviewed: Participant has at least a 1-year history of migraine (with or without aura) consistent with a diagnosis according to the International Classification of Headache Disorders, 3rd Edition.
   - Age of onset of migraines prior to 50 years of age
   - Migraine attacks, on average, lasting 4-72 hours if untreated
   - Per participant report, 4-14 migraine days per month within the last 3 months prior to the Screening Visit (a month is defined as 28 days)
   - Four to fourteen migraine days during the run-in period
6. Is currently managing migraines with ≥1 prescription acute treatment and/or prescription first or second-line preventive medications, as assessed by a physician.
7. Is the sole user of an iPhone with an iPhone operating system (iOS) 14 or later or a smartphone with an Android operating system (OS) 11 or later and is willing to download and use the Study App required by the protocol.
8. Is willing and able to receive SMS text messages and push messages on their smartphone.
9. Is the owner of, and has regular access to, an email address.
10. Has regular access to the Internet via cellular data plan and/or wifi.

### Exclusion Criteria

A participant will not be eligible for study entry if any of the physician-reviewed criteria are met:
1. History of basilar migraine or hemiplegic migraine.
2. Active chronic pain syndromes, such as fibromyalgia, chronic pelvic pain, or complex regional pain syndrome (CRPS).
3. Other pain syndromes (including trigeminal neuralgia), psychiatric conditions (such as major depressive episode, bipolar disorder, major depressive disorder, schizophrenia), dementia, or significant neurological disorders (other than migraine) that, in the Investigator's opinion, might interfere with study assessments.
4. History of, treatment for, or evidence of, alcohol or drug abuse within the past 12 months (48 weeks) or having met DSM-V criteria for any significant substance use disorder within the past 12 months (48 weeks) from the date of the screening visit.
5. History of use of analgesics (e.g., nonsteroidal anti-inflammatory drugs [NSAIDs] or acetaminophen, including opioids) or butalbital on ≥ 15 days per month during the 3 months (12 weeks) prior to the Screening Visit or during the run-in period.
6. Currently taking a prescription anti-calcitonin gene-related peptide (CGRP) for either episodic or chronic migraine.
7. Post-traumatic headache, persistent post-traumatic headache, or post-concussion syndrome.
8. Other significant episodic or chronic medical condition(s) that in the opinion of the Investigator, may confound the interpretation of findings to inform PDT development.
9. Failure to adhere with or inability to complete Study App inputs and onboarding activities during the run-in period. Participants who are not adherent during the run-in period are not eligible for study entry.
10. Previous enrollment in any digital therapeutics pilot or pivotal study for a migraine indication.
11. Participation in any other investigational clinical study while participating in this clinical study.

Test Product and Mode of Administration: Eligible participants will download and install the required Study App onto their own smartphone at the start of the run-in period. Participants will complete onboarding requirements during the run-in period and will be randomized to either CT-132 or Digital Control at the Baseline Visit (Day 1).

Study Duration: Participation in the study will last for approximately 19 weeks:
- Screening Period: Up to 2 weeks
- Run-In Period: 4 weeks
- Intervention Period: 12 weeks
- Follow-up Period: Up to 1 week

Sample Size: Approximately 558 participants are planned to be randomized in this study.

Statistical Analysis: Let µ1 and µ2 be the change from baseline in the MMD at the CT-132 and Digital Control arm, respectively. The null hypothesis for the primary endpoint is that the two are equal and the alternative hypothesis is that they are different.

An Analysis of Covariance (ANCOVA) model will be used to test this hypothesis, where the dependent variable is the change from baseline to Week 12 in MMD for each participant. A participant's baseline MMD will be the total number of migraine days recorded during the 28-day run-in period. The Week 12 MMD will be the total number of migraine days recorded over the previous 28 days (Weeks 9-12).

The model will adjust for the following covariates: treatment arm, baseline MMD, and gender. Analysis will include the modified Intent-to-Treat (mITT) analysis set. Multiple imputations under Missing At Random will be used to account for early termination.

### OBJECTIVES AND ENDPOINTS

The study objectives are to evaluate the efficacy and safety of CT-132 in reducing the number of MMDs, compared with a Digital Control, among late adolescents and adults with migraine.

### The study endpoints to support these objectives are listed in Table 1.

A participant's baseline MMD and MHD will be the total number of migraine days recorded during the 28-day run-in period. The Week 12 MMD and MHD will be the total number of migraine days recorded over the previous 28 days (Weeks 9-12).

**Table 1: Study Endpoints**

| **Primary Efficacy Endpoint** | |
|---|---|
| • Change from baseline in the number of MMD at Week 12 | |

| **Secondary Efficacy Endpoints** | |
|---|---|
| • Proportion of participants who have at least a 50% reduction from baseline in the mean number of MMD at Week 12 | |
| • Change from baseline in the number of MMD recorded over the previous 28 days at Week 4 and Week 8 | |
| • Reduction from baseline in the mean number of MNM over 12 weeks | |
| • Change in the average severity of headache from the run-in period to Weeks 9-12 | |
| • Change from baseline in the Migraine-Specific Quality-of-Life Questionnaire v2.1 (MSQ) at Week 4, Week 8, and Week 12 | |
| • Change from baseline in the Migraine Disability Assessment (MIDAS) at Week 4, Week 8, and Week 12 | |
| • Change in the use (frequency, dose, and type) of acute migraine medications from the run-in period to Weeks 9-12 | |

| **Exploratory Endpoints** | |
|---|---|
| • Key engagement metrics | |
| • Participant-reported information to include, for example, data: | |
| | • To evaluate health care utilization via the CHUM, by change from baseline at Week 4, Week 8, and Week 12 |
| | • To evaluate anxiety and depression via the GAD-7 and PHQ-8, respectively, by change from baseline at Week 4, Week 8, and Week 12 |
| | • To evaluate frequency of acute medication use at Week 4 and Week 8 in comparison to baseline |
| • Change from baseline in adherence to preventive medication as evaluated by the Voils DOSE-Nonadherence measure at Week 12 | |
| • Monthly headache days (MHD) | |
| | • Change from baseline in the number of MHD at Week 12 |
| | • Proportion of participants who have at least a 50% reduction from baseline in the mean number of MHD at Week 12 |
| | • Change from baseline in the number of MHD recorded over the previous 28 days at Week 4 and Week 8 |
| | • Reduction from baseline in the mean number of MHDs over 12 weeks |

| **Safety Endpoints** | |
|---|---|
| • Frequency and severity of AEs, serious AEs, and discontinuation from the study due to AEs | |
| • Frequency and severity of AEs related to the worsening of migraine | |

### Study Assessments

The following efficacy assessment scales are used in this trial at the times as provided in the SoA (Section 1.2). A description of the scales and the respective scoring algorithms for all endpoints will be provided in the Statistical Analysis Plan (SAP).

### Migraine Patient Journey Questionnaire

The Migraine Patient Journey Questionnaire is a 4-item Click-generated self-report questionnaire to characterize migraine history.

### Monthly Migraine Days (MMD)

Participants will record the number of days per month (defined as 28 days) a participant experiences migraine events within the Study App.

### Identify Chronic Migraine (ID-CM)

The Identify Chronic Migraine (ID-CM) is a 12-item self-report standardized assessment designed to assess presence of self-report migraine diagnosis and subsequently categorizes respondents as meeting diagnostic criteria for either episodic or chronic migraine.

### Migraine-Specific Quality-of-Life (MSQ)

The Migraine-Specific Quality-of-Life Questionnaire version 2.1 (MSQ) is a 14-item questionnaire that measures the impact of migraine across three essential aspects of a patient's health-related quality of life over the past 4 weeks.

### Migraine Disability Assessment Scale (MIDAS)

The Migraine Disability Assessment (MIDAS) questionnaire is a brief, self-report 7-item questionnaire designed to quantify headache-related disability over a 3-month period.

### Patient Health Questionnaire-8 (PHQ-8)

The Patient Health Questionnaire-8 (PHQ-8) is an 8-item self-report measure to establish depressive disorder diagnoses as well as grade depressive symptom severity.

### The Generalized Anxiety Disorder-7 (GAD-7)

The Generalized Anxiety Disorder-7 (GAD-7) is a 7-item self-report valid and efficient tool for screening for Generalized Anxiety Disorder and assessing its severity in clinical practice and research.

### Custom Healthcare Utilization Method (CHUM)

The Custom Healthcare Utilization Measure (CHUM) is an 8-item Click-generated self-report questionnaire that captures migraine-related healthcare visits across several settings in the previous 4 weeks.

### Voils DOSE-Nonadherence Measure

The Voils DOSE-Nonadherence measure is a two-domain scale designed to assess how many doses are missed and the reasons for missed doses. Various ways in which a patient can be nonadherent to their medications (e.g., missed doses, failing to fill prescriptions) can be measured.

### Patient Global Impression of Change (PGI-C)

The Patient Global Impression of Change (PGI-C) is a self-reported, 7-point scale depicting a patient's rating of overall improvement. PGI-C has been validated in a number of other episodic diseases against well-established measures of pain intensity, pain interference in daily life, and treatment effectiveness.

### Study App Engagement Assessments

### Key engagement metrics will be measured:

- Number of completed daily lessons out of total scheduled daily lessons
- Number of days a participant opens the Study App to engage beyond eDiary:
   - Where "Engagement beyond eDiary" means: any of the following actions done after a participant opens their eDiary for that day regardless of completion: lesson open, Decision Support tool open, or skill open
- Number of times user opens the Decision Support tool
- Number of times the user accesses skill practice from the skills tab
- Number of Study App opens per day on average

### Primary Endpoint

### The primary analysis will include the mITT set.

The primary endpoint is change from baseline in the number of MMD at Week 12. The primary endpoint will be analyzed using an ANCOVA model, where the dependent variable is the change from baseline to Week 12 in MMD for each participant. A participant's baseline MMD will be the total number of migraine days recorded during the 28-day run-in period. The Week 12 MMD will be the total number of migraine days recorded over the previous 28 days (Weeks 9-12). The model will adjust for the following covariates: treatment arm, baseline MMD, and gender. Multiple imputations under Missing At Random will be used to account for early termination. Least Squares Means (LSMEANS) for the difference in the change from baseline between the two treatment arms will be presented as well as the associated 95% confidence interval and two-sided p-value to test the null hypothesis of no difference. The study will be considered successful if the two-sided p-value will be ≤ 0.05.

### Sensitivity Analysis for Primary Endpoint

The primary analysis will be repeated for the ITT and PP analysis sets.

The SAP will define additional analyses to assess impact of missing data.

### Subgroups

The primary model will be repeated within each level of the following subgroups; however, the study may not be powered to show statistical significance for each of the subgroups:
- Gender (Males, Females)
- Age groups (18-21; 22-35; 36-64, 65+)
- Number of MMD at baseline (as reported using the run-in) (<8 days; ? 8 days)
- Subject's current prescription migraine medications at baseline (acute only; preventive L1 with or without acute; preventive L2 with or without acute medications)

### Secondary Endpoints

Analysis of secondary endpoints will include the mITT set.
- Proportion of participants that have at least 50% reduction from baseline in the mean number of MMD at Week 12 will be analyzed using logistics regression.
- Change from baseline in number of MMD recorded over the previous 28 days at Week 4 and to Week 8 will be analyzed in the same manner as the primary endpoint.
- Reduction from baseline in the mean number of MMD over 12 weeks will be analyzed in the same manner as the primary endpoint, but comparing the average MMD during the study period to the number of MMD recorded during the 28-day run-in period.
- Change from baseline in the severity of headache over 12 weeks will be analyzed in the same manner as the primary endpoint, but comparing average headache severity over Weeks 9-12 with average headache severity during the run-in period.
- Change from baseline in the Migraine-Specific Quality-of-Life Questionnaire v2.1 (MSQ) at Week 4, Week 8 and Week 12 will be analyzed using ANCOVA.
- Change from baseline in the Migraine Disability Assessment (MIDAS) at Week 4, Week 8, and Week 12 will be analyzed using ANCOVA.
- Change from baseline in the frequency of use of acute migraine medications in Weeks 9-12

The SAP will elaborate on the analysis of other endpoints, covariates and handling of missing data.

### Example 3 - Use of Calmness Assessment, Concentration Assessment, and Time Availability Assessment for Digital Therapeutics to Address Migraines

In one example, CT-132 mobile app (e.g., the application 120) will be given to individuals (e.g., in a manner as described above in Example 2) who have 4-14 migraine days in a month and on a medication for migraine. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about their level of calmness, difficulty in concentration, and immediate time availability. Based on the individual's reported levels of those 3 variables, the mobile app will recommend different energizing and calming strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 4 - Use of Calmness Assessment, Concentration Assessment, and Time Availability Assessment in Conjunction with Migraine States for Digital Therapeutics to Address Migraines

In one example, CT-132 mobile app (e.g., the application 120) will be given to individuals (e.g., in a manner as described above in Example 2) who have a number of migraine days in a month and on a medication for migraine. The decision support tool is an in-the-moment interactive method of recommending to individuals skills practices and strategies to help them feel better or behave around the migraine they may be experiencing, to help them decide what activity to do relevant to their migraine treatment. The decision support tool described herein will ask individuals questions about their migraine state plus level of calmness, difficulty in concentration, and immediate time availability. Based on the individual's reported levels of those 4 variables, the mobile app will recommend different skills practices. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 5 - Use of Assessed Characteristics for Digital Therapeutics to Address Migraines

In one example, CT-132 mobile app (e.g., the application 120) will be given to individuals (e.g., in a manner as described above in Example 2) who have 4-14 migraine days in a month and on a medication for migraine. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. Based on the individual's characteristics, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 6 - Use of Assessed Characteristics in Conjunction with Migraine States for Digital Therapeutics to Address Migraines

In one example, CT-132 mobile app (e.g., the application 120) will be given to individuals (e.g., in a manner as described above in Example 2) who have 4-14 migraine days in a month and on a medication for migraine. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool will also inquire about a migraine state of the user. Based on the individual's characteristics and migraine state, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 7 - Use of Assessed Characteristics in Conjunction with Migraine States and Calmness Assessment, Concentration Assessment, and Time Availability Assessment for Digital Therapeutics to Address Migraines

In one example, CT-132 mobile app (e.g., the application 120) will be given to individuals (e.g., in a manner as described above in Example 2) who have 4-14 migraine days in a month and on a medication for migraine. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool described herein will ask individuals questions about their migraine state plus level of calmness, difficulty in concentration, and immediate time availability. Based on the individual's characteristics, migraine state, calmness, difficulty in concentration, and time availability, the mobile app will recommend different strategies. It is expected that users will show a decrease in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app. Example 8 - Use of Assessed Characteristics in Conjunction with Calmness Assessment, Concentration Assessment, and Time Availability Assessment for Digital Therapeutics to Address Migraines

In one example, CT-132 mobile app (e.g., the application 120) will be given to individuals (e.g., in a manner as described above in Example 2) who have 4-14 migraine days in a month and on a medication for migraine. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool described herein will ask individuals questions about their level of calmness, difficulty in concentration, and immediate time availability. Based on the individual's characteristics, calmness, difficulty in concentration, and time availability, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 9 - Use of Calmness Assessment, Concentration Assessment, and Time Availability Assessment for Digital Therapeutics to Address Migraines for Users Not Taking Medication

In one example, CT-132 mobile app (e.g., the application 120) will be given to individuals (e.g., in a manner as described above in Example 2) who have 4-14 migraine days in a month. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about their level of calmness, difficulty in concentration, and immediate time availability. Based on the individual's reported levels of those 3 variables, the mobile app will recommend different energizing and calming strategies. It is expected that users will show a decrease in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 10 - Use of Calmness Assessment, Concentration Assessment, and Time Availability Assessment in Conjunction with Migraine States for Digital Therapeutics to Address Migraines for Users Not Taking Medication

In one example, CT-132 mobile app (e.g., the application 120) will be given to individuals (e.g., in a manner as described above in Example 2) who have a number of migraine days in a month. The decision support tool is an in-the-moment interactive method of recommending to individuals skills practices and strategies to help them feel better or behave around the migraine they may be experiencing, to help them decide what activity to do relevant to their migraine treatment. The decision support tool described herein will ask individuals questions about their migraine state plus level of calmness, difficulty in concentration, and immediate time availability. Based on the individual's reported levels of those 4 variables, the mobile app will recommend different skills practices. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 11 - Use of Assessed Characteristics for Digital Therapeutics to Address Migraines for Users Not on Medication

In one example, CT-132 mobile app (e.g., the application 120) will be given to individuals (e.g., in a manner as described above in Example 2) who have 4-14 migraine days in a month. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. Based on the individual's characteristics, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 12 - Use of Assessed Characteristics in Conjunction with Migraine States for Digital Therapeutics to Address Migraines for Users Not on Medication

In one example, CT-132 mobile app (e.g., the application 120) will be given to individuals (e.g., in a manner as described above in Example 2) who have 4-14 migraine days in a month. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool will also inquire about a migraine state of the user. Based on the individual's characteristics and migraine state, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### Example 13 - Use of Assessed Characteristics in Conjunction with Migraine States and Calmness Assessment, Concentration Assessment, and Time Availability Assessment for Digital Therapeutics to Address Migraines for Users Not on Medication

In one example, CT-132 mobile app (e.g., the application 120) will be given to individuals (e.g., in a manner as described above in Example 2) who have 4-14 migraine days in a month. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool described herein will ask individuals questions about their migraine state plus level of calmness, difficulty in concentration, and immediate time availability. Based on the individual's characteristics, migraine state, calmness, difficulty in concentration, and time availability, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app. Example 14 - Use of Assessed Characteristics in Conjunction with Migraine States and Calmness Assessment, Concentration Assessment, and Time Availability Assessment for Digital Therapeutics to Address Migraines for Users Not on Medication

In one example, CT-132 mobile app (e.g., the application 120) will be given to individuals (e.g., in a manner as described above in Example 2) who have 4-14 migraine days in a month. The treatment will be 12 weeks long. The decision support tool is an in-the-moment interactive method of recommending to individuals strategies to help them feel better or how to behave around the migraine they may be experiencing, to help them decide what activity to do relevant to their migraine treatment. The decision support tool described herein will ask individuals questions about various characteristics to be assessed, such as a migraine symptom, a vocal biomarker, an anxiety level, an engagement level, and mood, among others. The decision support tool described herein will ask individuals questions about their level of calmness, difficulty in concentration, and immediate time availability. Based on the individual's characteristics, calmness, difficulty in concentration, and time availability, the mobile app will recommend different strategies. It is expected that users will show a decrease (or an improvement) in the mean number of monthly migraine days (MDD), pain catastrophizing scale (PCS) scores, Difficulties in Emotion Regulation Scale Short Form (DERS-SF), Generalized Anxiety Disorder 7-item (GAD-7), Identify Chronic Migraine (ID-CM), Patient Health Questionnaire-8 (PHQ-8), or Self-Efficacy for Managing Chronic Disease 6-Item Scale (SES6C), among others, over the course of using the CT-132 mobile app.

### C. Network and Computing Environment

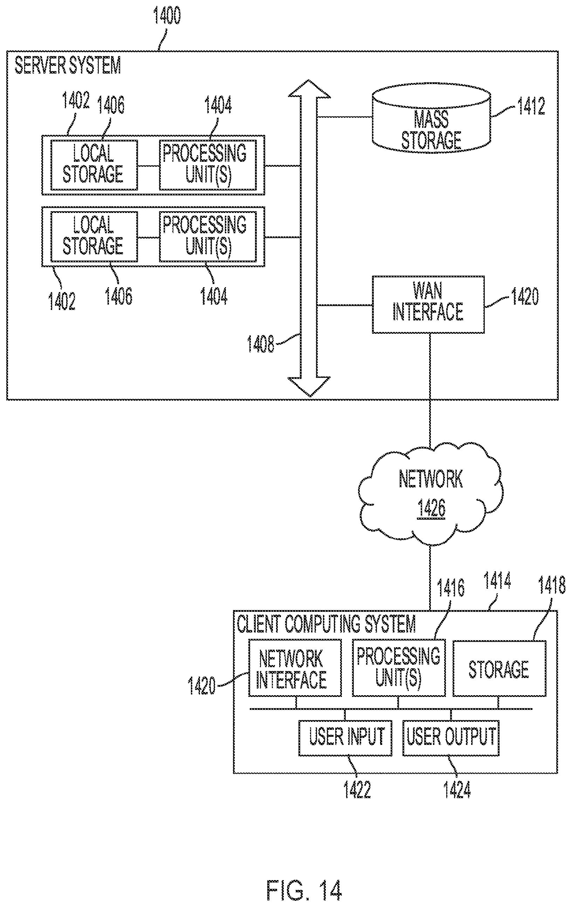

Various operations described herein can be implemented on computer systems. FIG. 14 shows a simplified block diagram of a representative server system 1400, client computer system 1414, and network 1426 usable to implement certain embodiments of the present disclosure. In various embodiments, server system 1400 or similar systems can implement services or servers described herein or portions thereof. Client computer system 1414 or similar systems can implement clients described herein. The system 100 described herein can be similar to the server system 1400. Server system 1400 can have a modular design that incorporates a number of modules 1402 (e.g., blades in a blade server embodiment); while two modules 1402 are shown, any number can be provided. Each module 1402 can include processing unit(s) 1404 and local storage 1406.

Processing unit(s) 1404 can include a single processor, which can have one or more cores, or multiple processors. In some embodiments, processing unit(s) 1404 can include a general-purpose primary processor as well as one or more special-purpose co-processors such as graphics processors, digital signal processors, or the like. In some embodiments, some or all processing units 1404 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 1404 can execute instructions stored in local storage 1406. Any type of processors in any combination can be included in processing unit(s) 1404.

Local storage 1406 can include volatile storage media (e.g., DRAM, SRAM, SDRAM, or the like) and/or non-volatile storage media (e.g., magnetic or optical disk, flash memory, or the like). Storage media incorporated in local storage 1406 can be fixed, removable, or upgradeable as desired. Local storage 1406 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 1404 need at runtime. The ROM can store static data and instructions that are needed by processing unit(s) 1404. The permanent storage device can be a non-volatile read-and-write memory device that can store instructions and data even when module 1402 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 1406 can store one or more software programs to be executed by processing unit(s) 1404, such as an operating system and/or programs implementing various server functions such as functions of the system 100 or any other system described herein, or any other server(s) associated with system 100 or any other system described herein.

"Software" refers generally to sequences of instructions that, when executed by processing unit(s) 1404, cause server system 1400 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 1404. Software can be implemented as a single program or a collection of separate programs or program modules that interact as desired. From local storage 1406 (or non-local storage described below), processing unit(s) 1404 can retrieve program instructions to execute and data to process in order to execute various operations described above.

In some server systems 1400, multiple modules 1402 can be interconnected via a bus or other interconnect 1408, forming a local area network that supports communication between modules 1402 and other components of server system 1400. Interconnect 1408 can be implemented using various technologies, including server racks, hubs, routers, etc.

A wide area network (WAN) interface 1410 can provide data communication capability between the local area network (e.g., through the interconnect 1408) and the network 1426, such as the Internet. Other technologies can be used to communicatively couple the server system with the network 1426, including wired (e.g., Ethernet, IEEE 802.3 standards) and/or wireless technologies (e.g., Wi-Fi, IEEE 802.11 standards).

In some embodiments, local storage 1406 is intended to provide working memory for processing unit(s) 1404, providing fast access to programs and/or data to be processed while reducing traffic on interconnect 1408. Storage for larger quantities of data can be provided on the local area network by one or more mass storage subsystems 1412 that can be connected to interconnect 1408. Mass storage subsystem 1412 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 1412. In some embodiments, additional data storage resources may be accessible via WAN interface 1410 (potentially with increased latency).

Server system 1400 can operate in response to requests received via WAN interface 1410. For example, one of modules 1402 can implement a supervisory function and assign discrete tasks to other modules 1402 in response to received requests. Work allocation techniques can be used. As requests are processed, results can be returned to the requester via WAN interface 1410. Such operation can generally be automated. Further, in some embodiments, WAN interface 1410 can connect multiple server systems 1400 to each other, providing scalable systems capable of managing high volumes of activity. Other techniques for managing server systems and server farms (collections of server systems that cooperate) can be used, including dynamic resource allocation and reallocation.

Server system 1400 can interact with various user-owned or user-operated devices via a wide-area network such as the Internet. An example of a user-operated device is shown in FIG. 14 as client computing system 1414. Client computing system 1414 can be implemented, for example, as a consumer device such as a smartphone, other mobile phone, tablet computer, wearable computing device (e.g., smart watch, eyeglasses), desktop computer, laptop computer, and so on.

For example, client computing system 1414 can communicate via WAN interface 1410. Client computing system 1414 can include computer components such as processing unit(s) 1416, storage device 1418, network interface 1420, user input device 1422, and user output device 1424. Client computing system 1414 can be a computing device implemented in a variety of form factors, such as a desktop computer, laptop computer, tablet computer, smartphone, other mobile computing device, wearable computing device, or the like.

Processing unit 1416 and storage device 1418 can be similar to processing unit(s) 1404 and local storage 1406 described above. Suitable devices can be selected based on the demands to be placed on client computing system 1414; for example, client computing system 1414 can be implemented as a "thin" client with limited processing capability or as a highpowered computing device. Client computing system 1414 can be provisioned with program code executable by processing unit(s) 1416 to enable various interactions with server system 1400.

Network interface 1420 can provide a connection to the network 1426, such as a wide area network (e.g., the Internet) to which WAN interface 1410 of server system 1400 is also connected. In various embodiments, network interface 1420 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 1422 can include any device (or devices) via which a user can provide signals to client computing system 1414; client computing system 1414 can interpret the signals as indicative of particular user requests or information. In various embodiments, user input device 1422 can include any or all of a keyboard, touch pad, touch screen, mouse or other pointing device, scroll wheel, click wheel, dial, button, switch, keypad, microphone, and so on.

User output device 1424 can include any device via which client computing system 1414 can provide information to a user. For example, user output device 1424 can include display-to-display images generated by or delivered to client computing system 1414. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 1424 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 1404 and 1416 can provide various functionality for server system 1400 and client computing system 1414, including any of the functionality described herein as being performed by a server or client, or other functionality.

It will be appreciated that server system 1400 and client computing system 1414 are illustrative and that variations and modifications are possible. Computer systems used in connection with embodiments of the present disclosure can have other capabilities not specifically described here. Further, while server system 1400 and client computing system 1414 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Various aspects of this disclosure are set out in the following enumerated clauses:
1. A system for dynamically providing messages, comprising:
   a computing system having one or more processors coupled with memory, configured to:
   maintain, on a database, an identification of a plurality of activities to address migraines in a user at risk thereof, each of the plurality of activities having a plurality of messages to be presented in accordance with a rule;
   receive, via a user interface, an indication identifying a migraine state of the user as having one of: an onset, a presence, or an absence of the migraine;
   select, based on the migraine state of the user identified in the response, an activity from the plurality of activities to provide to the user;
   identify, in accordance with the rule for the activity, a message from the plurality of messages; and
   cause the message identifying the activity to be presented via the user interface.
2. The system of clause 1, wherein the computing system is further configured to:
   maintain a timer identifying a time elapsed since a presentation of a prior message via the user interface; and
   provide, responsive to the time elapsing a threshold, the user interface prompting for the indication of the migraine state of the user.
3. The system of clause 1, wherein the computing system is further configured to provide, in accordance with a schedule, the user interface prompting for the indication of the migraine state of the user.
4. The system of clause 1, wherein the computing system is further configured to select, based on the migraine state of the user, the activity from the plurality of activities, wherein the plurality of activities comprises (i) a first activity having the plurality of messages associated with preventing the onset of the migraine, (ii) a second activity having the plurality of messages associated with alleviating the presence of the migraine, or (iii) a third activity having the plurality of messages associated with maintaining the absence of the migraine.
5. The system of clause 1, wherein the computing system is further configured to:
   receive, via the user interface, a response identifying an activity performed by the user in response to presentation of the message of the activity; and
   update, on the database, a profile of the user to include the response identifying the activity performed by the user.
6. The system of clause 1, wherein the computing system is further configured to identify the message from the plurality of messages for the activity in accordance with the rule based on at least one of (i) a selection of a second activity for a prior message, (ii) a response by the user to a presentation of the prior message, (iii) a number of activities performed by the user, or (iv) a taking of a medication by the user.
7. The system of clause 1, wherein the computing system is further configured to identify, in accordance with the rule, a group of messages from the plurality of messages to present within a time duration.
8. The system of clause 1, wherein the computing system is further configured to select the activity from the plurality of activities based on at least one of (i) a number of occurrences of the migraine in the user, (ii) a duration of a prior occurrence of the migraine, or (iii) a taking of a medication by the user.
9. The system of clause 1, wherein the computing system is further configured to provide, to an application on a user device, an instruction for presentation of the message to the user associated with the application.
10. The system of clause 1, wherein the computing system is further configured to:
   identify, from the indication, a plurality of emotional states including a calmness, a time availability, and a mental stamina of the user;
   select, based on the plurality of emotional states and the migraine state, the activity from the plurality of activities.
11. The system of clause 1, wherein the computing system is further configured to:
   identify, from the indication, at least one characteristic of the user from a plurality of characteristics to be assessed, the plurality of characteristics including one or more of a migraine symptom, a vocal biomarker, a mood, and an engagement level;
   select, based on the at least one characteristic and the migraine state, the activity from the plurality of activities.
12. The system of clause 1, wherein the user is on a medication to address the migraine at least in partial concurrence with the activity.
13. A method of dynamically providing messages, comprising:
   maintaining, by the computing system, on a database, an identification of a plurality of activities to address migraines in a user at risk thereof, each of the plurality of activities having a plurality of messages to be presented in accordance with a rule;
   receiving by the computing system, via a user interface, an indication identifying a migraine state of the user as having one of: an onset, a presence, or an absence of the migraine;
   selecting, by the computing system, based on the migraine state of the user identified in the response, a activity from the plurality of activities to provide to the user;
   identifying, by the computing system, in accordance with the rule for the activity, a message from the plurality of messages; and
   presenting, by the computing system, via the user interface, the message of the activity selected from the plurality of activities.
14. The method of clause 13, further comprising:
   maintaining, by the computing system, a timer identifying a time elapsed since a presentation of a prior message via the user interface; and
   providing, by the computing system, responsive to the time elapsing a threshold, the user interface prompting for the indication of the migraine state of the user.
15. The method of clause 13, further comprising providing, by the computing system, in accordance with a schedule, the user interface prompting for the indication of the migraine state of the user.
16. The method of clause 13, wherein selecting the activity further comprises selecting, based on the migraine state of the user, the activity from the plurality of activities, wherein the plurality of activities comprises (i) a first activity having the plurality of messages associated with preventing the onset of the migraine, (ii) a second activity having the plurality of messages associated with alleviating the presence of the migraine, or (iii) a third activity having the plurality of messages associated with maintaining the absence of the migraine.
17. The method of clause 13, further comprising:
   receiving, by the computing system, via the user interface, a response identifying an activity performed by the user in response to presentation of the message of the activity; and
   updating, by the computing system, on the database, a profile of the user to include the response identifying the activity performed by the user.
18. The method of clause 13, wherein identifying further comprises identifying message from the plurality of messages for the activity in accordance with the rule based on at least one of (i) a selection of a second activity for a prior message, (ii) a response by the user to a presentation of the prior message, (iii) a number of activities performed by the user, or (iv) a taking of a medication by the user.
19. The method of clause 13, wherein identifying further comprises identifying, accordance with the rule, a subset of messages from the plurality of messages to present within a time duration.
20. The method of clause 13, wherein selecting further comprises selecting the activity from the plurality of activities at least one of (i) a number of occurrences of the migraine in the user, (ii) a duration of a prior occurrence of the migraine, or (iii) a taking of a medication by the user.
21. The method of clause 13, further comprising providing, by the computing system, to an application on a user device, an instruction for presentation of the message to the user associated with the application.
22. The method of clause 13, further comprising:
   identifying, by the computing system, from the indication , a plurality of emotional states including a calmness, a time availability, and a mental stamina of the user;
   wherein selecting the activity further comprises selecting, based on the plurality of emotional states and the migraine state, the activity from the plurality of activities.
23. The method of clause 13, further comprising:
   identifying, by the computing system, at least one characteristic of the user from a plurality of characteristics to be assessed, the plurality of characteristics including one or more of a migraine symptom, a vocal biomarker, a mood, and an engagement level;
   wherein selecting the activity further comprising selecting, based on the plurality of characteristics and the migraine state, the activity from the plurality of activities.
24. The method of clause 13, wherein the user is on a medication to address the migraine at least in partial concurrence with the activity.
25. A system for dynamically providing messages, comprising:
   a computing system having one or more processors coupled with memory, configured to:
   maintain, on a database, an identification of a plurality of activities to address migraines in a user at risk thereof, each of the plurality of activities having a plurality of messages to be presented in accordance with a rule;
   receive, via a user interface, an indication identifying at least one of a plurality of emotional states of the user;
   select, based on the indication, an activity from the plurality of activities to provide to the user;
   identify, in accordance with the rule for the activity, a message from the plurality of messages; and
   cause the message identifying the activity to be presented via the user interface.
26. The system of clause 25, wherein the computing system is further configured to:
   identify, from the indication, the plurality of emotional states including a calmness, a time availability, and a mental stamina of the user;
   select, based on the plurality of emotional states, the activity from the plurality of activities.
27. The system of clause 25, wherein the computing system is further configured to:
   identify, from the indication, at least one of the plurality of emotional states as the time availability identifying an amount of available time for the user to perform at least one of the plurality of activities; and
   select, based on a comparison of the amount of available time with a threshold time, the activity from the plurality of activities, wherein the plurality of activities comprises (i) a first activity associated with the amount of available time exceeding the threshold time and (ii) a second activity associated with the amount of available time not exceeding the threshold time.
28. The system of clause 25, wherein the computing system is further configured to:
   identify, from the indication, a migraine state of the user as having one of: an onset, a presence, or an absence of a migraine; and
   select, based on the migraine state and the plurality of emotional states of the user, the activity from the plurality of activities.
29. The system of clause 25, wherein the computing system is further configured to:
   identify, from the indication, at least one characteristic of the user from a plurality of characteristics to be assessed, the plurality of characteristics including one or more of a migraine symptom, a vocal biomarker, a mood, and an engagement level;
   select, based on the at least one characteristic, the activity from the plurality of activities.
30. The system of clause 25, wherein the computing system is further configured to:
   identify, from the indication, (i) at least one characteristic of the user from a plurality of characteristics to be assessed and (ii) a migraine state of the user; and
   select the activity based on a combination of the plurality of emotional states, the at least one characteristic, and the migraine state.
31. The system of clause 25, wherein the computing system is further configured to identify, in accordance with the rule, a group of messages from the plurality of messages to present within a time duration.
32. The system of clause 25, wherein the computing system is further configured to:
   maintain a timer identifying a time elapsed since a presentation of a prior message via the user interface; and
   provide, responsive to the time elapsing a threshold, the user interface prompting for the indication of the plurality of emotional states of the user.
33. The system of clause 25, wherein the computing system is further configured to provide, in accordance with a schedule, the user interface prompting for the indication of the plurality of emotional states of the user.
34. The system of clause 25, wherein the computing system is further configured to:
   receive, via the user interface, a response identifying an activity performed by the user in response to presentation of the message of the activity; and
   update, on the database, the profile to include the response identifying the activity performed by the user.
35. The system of clause 25, wherein the user is on a medication to address the migraine at least in partial concurrence with the activity.
36. A method of dynamically providing messages, comprising:
   maintaining, by a computing system, on a database, an identification of a plurality of activities to address migraines in a user at risk thereof for the session, each of the plurality of activities having a plurality of messages to be presented in accordance with a rule;
   receiving, by the computing system, via a user interface, an indication identifying at least one of a plurality of emotional states of the user;
   selecting, by the computing system, based on the indication, an activity from the plurality of activities to provide to the user;
   identifying, by the computing system, in accordance with the rule for the activity, a message from the plurality of messages; and
   presenting, by the computing system, via the user interface, the message of the activity selected from the plurality of activities.
37. The method of clause 36, further comprising identifying, by the computing system, from the indication, the plurality of emotional states including a calmness, a time availability, and a mental stamina of the user; and
   wherein selecting the activity further comprises selecting, based on the plurality of emotional states, the activity from the plurality of activities.
38. The method of clause 36, further comprising identifying, by the computing system, from the indication, at least one of the plurality of emotional states as the time availability identifying an amount of available time for the user to perform at least one of the plurality of activities; and
   wherein selecting the activity further comprises selecting the activity, based on a comparison of the amount of available time with a threshold time, the activity from the plurality of activities, wherein the plurality of activities comprises (i) a first activity associated with the amount of available time exceeding the threshold time and (ii) a second activity associated with the amount of available time not exceeding the threshold time.
39. The method of clause 36, further comprising identifying, by the computing system, from the indication, at least one migraine state of the user as having one of: an onset, a presence, or an absence of a migraine; and
   wherein selecting the activity further comprises selecting, based on the migraine state and the plurality of emotional states of the user.
40. The method of clause 36, further comprising identifying, by the computing system, from the indication, at least one characteristic of the user from a plurality of characteristics to be assessed, the plurality of characteristics including one or more of a migraine symptom, a vocal biomarker, a mood, and an engagement level;
   wherein selecting the activity further comprises selecting, based on the at least one of characteristic.
41. The method of clause 36, further comprising identifying, by the computing system, from the indication, (i) at least one characteristic of the user from a plurality of characteristics to be assessed and (ii) a migraine state of the user; and
   wherein selecting the activity further comprises selecting the activity based on a combination of the plurality of emotional states, the at least one characteristic, and the migraine state.
42. The method of clause 36, further comprising identifying, by the computing system, in accordance with the rule, a group of messages from the plurality of messages to present within a time duration.
43. The method of clause 36, further comprising:
   maintaining, by the computing system, a timer identifying a time elapsed since a presentation of a prior message via the user interface; and
   providing, by the computing system, responsive to the time elapsing a threshold, the user interface prompting for the indication of the plurality of emotional states of the user.
44. The method of clause 36, further comprising providing, by the computing system, in accordance with a schedule, the user interface prompting for the indication of the state of the user.
45. The method of clause 36, further comprising:
   receiving, by the computing system, via the user interface, a response identifying an activity performed by the user in response to presentation of the message of the activity; and
   updating, by the computing system, on the database, the profile to include the response identifying the activity performed by the user.
46. The method of clause 36, wherein the user is on a medication to address the migraine at least in partial concurrence with the activity.
47. A method of reducing a number of migraine days in a user, comprising:
   presenting, by a computing device, to the user at risk of a migraine at a first time instance, a first message identifying a first activity of a plurality of activities, wherein the first activity is selected from the plurality of activities based on a first indication of a first migraine state of the user as one of a plurality of migraine states comprising one or more of an onset, a presence, or an absence of the migraine;
   obtaining, by the computing device, a first metric associated with the user prior to the first activity;
   repeating, by the computing device, to the user at a plurality of second time instances, a presentation of a respective second message identifying a second activity of the plurality of activities, the second activity is selected from the plurality of activities based on a second indication of a second migraine state of the user as one of the plurality of migraine states; and
   obtaining, by the computing device, a second metric associated with the user after the second plurality of time instances,
   wherein the user shows reduction in the number of migraine days, when the second metric is lower than the first metric from the first time instance.
48. The method of clause 47, wherein the plurality of activities comprises (i) a first activity having the plurality of messages associated with preventing the onset of the migraine, (ii) a second activity having the plurality of messages associated with alleviating the presence of the migraine, or (iii) a third activity having the plurality of messages associated with maintaining the absence of the migraine.
49. The method of clause 47, wherein the first metric at the first time instance comprises a first pain catastrophizing scale (PCS) score at a baseline and wherein the second metric at a last of the plurality of second time instances comprises a second PCS.
50. The method of clause 47, wherein the first metric at the first time instance comprises a first PCS score at a baseline and wherein the second metric comprises a second PCS after at least one of the plurality of second time instances.
51. The method of clause 47, wherein the first metric at the first time instance comprises a first number of monthly migraine days (MDDs) at a baseline and wherein the second metric at a last of the plurality of second time instances comprises a second number of MDDs.
52. The method of clause 47, wherein the first metric at the first time instance comprises a first number of MDDs at a baseline and wherein the second metric comprises a second number MDDs after at least one of the plurality of second time instances.
53. The method of clause 47, wherein the migraine comprises at least one of a complicated migraine, a common migraine, a silent migraine, a hemiplegic migraine, or a retinal migraine.
54. The method of clause 47, wherein the user is on a medication to address the migraine at least in partial concurrence over at least one of the first time instance or the plurality of second time instances, wherein the medication comprises at least one of a triptan, an ibuprofen, an ergot, or a calcitonin gene-related peptide (CGRP) inhibitor.
55. A method of reducing a number of migraine days in a user, comprising:
   presenting, by a computing device, to the user at risk of a migraine at a first time instance, a first message identifying a first activity of a plurality of activities, wherein the first activity is selected from the plurality of activities based on a first indication of a first plurality of emotional states for the user;
   obtaining, by the computing device, a first metric associated with the user prior to the first activity;
   repeating, by the computing device, to the user at a plurality of second time instances, a presentation of a respective second message identifying a second activity of the plurality of activities, the second activity is selected from the plurality of activities based on a second indication of a second plurality of emotional states of the user; and
   obtaining, by the computing device, a second metric associated with the user after the second plurality of time instances,
   wherein the user shows reduction in the number of migraine days, when the second metric is lower than the first metric from the first time instance.
56. The method of clause 55, wherein the first plurality of emotional states and the second plurality of emotional states each comprise a calmness, a mental stamina, and a time availability of the user.
57. The method of clause 55, wherein at least one of the first emotional state or the second emotional state of the user identifies the time availability of the user; and
   wherein the plurality of activities includes (i) an activity associated with the time availability exceeding a threshold and (ii) an activity associated with the time availability not exceeding the threshold.
58. The method of clause 55, wherein the first metric at the first time instance comprises a first pain catastrophizing scale (PCS) score at a baseline and wherein the second metric at a last of the plurality of second time instances comprises a second PCS.
59. The method of clause 55, wherein the first metric at the first time instance comprises a first PCS score at a baseline and wherein the second metric comprises a second PCS after at least one of the plurality of second time instances.
60. The method of clause 55, wherein the first metric at the first time instance comprises a first number of monthly migraine days (MDDs) at a baseline and wherein the second metric at a last of the plurality of second time instances comprises a second number of MDDs.
61. The method of clause 55, wherein the first metric at the first time instance comprises a first number of MDDs at a baseline and wherein the second metric comprises a second number MDDs after at least one of the plurality of second time instances.
62. The method of clause 55, wherein the migraine comprises at least one of a complicated migraine, a common migraine, a silent migraine, a hemiplegic migraine, or a retinal migraine.
63. The method of clause 55, wherein the user is on a medication to address the migraine at least in partial concurrence over at least one of the first time instance or the plurality of second time instances, wherein the medication comprises at least one of a triptan, an ibuprofen, an ergot, or a calcitonin gene-related peptide (CGRP) inhibitor.
64. A method of reducing a number of migraine days in a user, comprising:
   presenting, by a computing device, to the user at risk of a migraine at a first time instance, a first message identifying a first activity of a plurality of activities, wherein the first activity is selected from the plurality of activities based on a first indication of a first migraine state and a first plurality of emotional states;
   obtaining, by the computing device, a first metric associated with the user prior to the first activity;
   repeating, by the computing device, to the user at a plurality of second time instances, a presentation of a respective second message identifying a second activity of the plurality of activities, the second activity is selected from the plurality of activities based on a second indication of a second migraine state and a second plurality of emotional states; and
   obtaining, by the computing device, a second metric associated with the user after the second plurality of time instances,
   wherein the user shows reduction in the number of migraine days, when the second metric is lower than the first metric from the first time instance.
65. The method of clause 64, wherein the first migraine state and the second migraine state further comprises one or more of an onset, a presence, or an absence of the migraine.
66. The method of clause 64, wherein the first plurality of emotional states and the second plurality of emotional states each comprise one or more of a calmness, a mental stamina, and a time availability of the user.
67. The method of clause 64, wherein the first indication or the second indication further identifies a characteristic of the user from a plurality of characteristics to be assessed, the plurality of characteristics including one or more of a migraine symptom, a vocal biomarker, a mood, and an engagement level to be used to select the first activity or the second activity.
68. The method of clause 64, wherein the first metric at the first time instance comprises a first pain catastrophizing scale (PCS) score at a baseline and wherein the second metric at a last of the plurality of second time instances comprises a second PCS.
69. The method of clause 64, wherein the first metric at the first time instance comprises a first PCS score at a baseline and wherein the second metric comprises a second PCS after at least one of the plurality of second time instances.
70. The method of clause 64, wherein the first metric at the first time instance comprises a first number of monthly migraine days (MDDs) at a baseline and wherein the second metric at a last of the plurality of second time instances comprises a second number of MDDs.
71. The method of clause 64, wherein the first metric at the first time instance comprises a first number of MDDs at a baseline and wherein the second metric comprises a second number MDDs after at least one of the plurality of second time instances.
72. The method of clause 64, wherein the migraine comprises at least one of a complicated migraine, a common migraine, a silent migraine, a hemiplegic migraine, or a retinal migraine.
73. The method of clause 64, wherein the user is on a medication to address the migraine at least in partial concurrence over at least one of the first time instance or the plurality of second time instances, wherein the medication comprises at least one of a triptan, an ibuprofen, an ergot, or a calcitonin gene-related peptide (CGRP) inhibitor.

Thus, although the disclosure has been described with respect to specific embodiments, it will be appreciated that the disclosure is intended to cover all modifications and equivalents within the scope of the following claims.

## Claims

1. A method of reducing a number of migraine and/or headache days in a user comprising:
presenting, by a computing system, to the user at risk of a migraine or headache at a first time instance, a first message identifying a first activity of a plurality of activities, wherein the first activity is selected from the plurality of activities based on a first indication of:
(i) a first migraine or headache state of the user, and/or
(ii) at least one first emotional state for the user;
obtaining, by the computing system, a first metric associated with the user prior to the first activity;
repeating, by the computing system, to the user at a plurality of subsequent time instances, a presentation of a respective subsequent message identifying a subsequent activity of the plurality of activities, the subsequent activity is selected from the plurality of activities based on a subsequent indication of:
(i) a subsequent migraine or headache state of the user, and/or
(ii) at least one subsequent emotional state of the user; and
obtaining, by the computing system, a second metric associated with the user after the plurality of subsequent time instances,
wherein the user shows reduction in the number of migraine or headache days, when the second metric is lower than the first metric from the first time instance.

2. The method of claim 1, wherein the first metric at the first time instance comprises a first pain catastrophizing scale (PCS) score at a baseline and wherein the second metric at a last of the plurality of subsequent time instances comprises a subsequent PCS score.

3. The method of claim **1,** wherein the first metric at the first time instance comprises a first PCS score at a baseline and wherein the second metric comprises a subsequent PCS after at least one of the plurality of subsequent time instances.

4. The method of claim 1, wherein the first metric at the first time instance comprises a first number of monthly migraine days (MDDs) or monthly headache days (MHDs) at a baseline and wherein the second metric at a last of the plurality of subsequent time instances comprises a second number of MDDs or MHDs.

5. The method of claim 1, wherein the first metric at the first time instance comprises a first number of MDDs or MHDs at a baseline and wherein the second metric comprises a second number MDDs or MHDs after at least one of the plurality of subsequent time instances.

6. The method of claim 1, wherein the first migraine or headache state and the subsequent migraine or headache state comprises one or more of an onset, a presence, or an absence of the migraine or headache.

7. The method of claim 1, wherein the at least one first emotional state and the at least one subsequent emotional state each comprise one or more of a calmness, a mental stamina, and a time availability of the user.

8. The method of claim 1, wherein the first indication or the subsequent indication further identifies a characteristic of the user from a plurality of characteristics to be assessed, the plurality of characteristics including one or more of a migraine symptom, a vocal biomarker, a mood, and an engagement level to be used to select the first activity or the subsequent activity.

9. The method of claim 1, further comprising:
maintaining, by the computing system, a timer identifying a time elapsed since a presentation of a prior message; and
provide, responsive to the time elapsing a threshold, a user interface prompting for the first indication and/or the subsequent indication.

10. The method of claim 1, further comprising providing, in accordance with a schedule, a prompting for the first indication and/or the subsequent indication.

11. The method of claim 1, wherein the computing system is further configured to:
receiving, by the computing system, a response identifying an activity performed by the user in response to presentation of the first message and/or the subsequent message of the activity; and
updating, by the computing system, on a database, a profile of the user to include the response identifying the activity performed by the user.

12. The method of claim 1, wherein the migraine comprises at least one of a complicated migraine, a common migraine, a silent migraine, a hemiplegic migraine, or a retinal migraine, and/or wherein the headache comprises an icepick headache or cluster headache.

13. The method of claim 1, wherein the user is on a medication to address the migraine or headache at least in partial concurrence with the activity, and optionally wherein the medication comprises at least one of a triptan, an ibuprofen, an ergot, or a calcitonin gene-related peptide (CGRP) inhibitor.

14. A system comprising a processor, and a storage medium storing instructions, which when executed by the processor, causes the system to carry out the method of any one of claims 1-13.

15. A machine-readable medium carrying machine readable instructions, which when executed by one or more processors, causes the system to carry out the method of any one of claims 1-13.
